# EUROPEAN PATENT APPLICATION

(11) **EP 4 299 738 A1**
(43) Date of publication of application: **03.01.2024**
(21) Application number: 22759830.7
(22) Date of filing: 25.02.2022
(51) Int. Cl.: C12N 15/09, A01H 1/00, A01H 5/00, A01H 5/10, C07K 19/00, C12N 5/10, C12N 9/16, C12N 9/24

(54) **SEMI-RATIONAL GENOME EVOLUTION ENGINEERING METHOD FOR PLANTS**

(30) Priority: 26.02.2021 JP 2021030805
(71) Applicant: National University Corporation Kobe University, Kobe-shi, Hyogo 657-8501 (JP)
(72) Inventor: NISHIDA, Keiji, Kobe-shi, Hyogo 657-8501 (JP); SHIMATANI, Zenpei, Kobe-shi, Hyogo 657-8501 (JP); FUJIKURA, Ushio, Kobe-shi, Hyogo 657-8501 (JP)
(74) Representative: Weickmann & Weickmann PartmbB
(86) International application number: PCT/JP2022/008035
(87) International publication number: WO 2022/181796

(57) **Abstract**

The present disclosure provides a method for site-specific modification of DNA molecules that can be isolated as individual mutant clones.

According to the present disclosure, a method for producing a plant cell modified at a targeted site of a double-stranded DNA, including (i) a step of providing a plant cell comprising the double-stranded DNA of interest, (ii) a step of providing a complex in which a nucleic acid sequence-recognizing module that specifically binds to a target nucleotide sequence in the double-stranded DNA and a DNA glycosylase with sufficiently low reactivity with the double-stranded DNA are bound, (iii) a step of placing the complex in a condition under which the plant cell is transfected, (iv) a step of placing the transfected plant cell in a condition that induces modification of the targeted site, without cleaving at least one strand of the double-stranded DNA in the targeted site, and (v) a step of selecting a cell into which the complex has been introduced and/or a cell into which the modification has been introduced is provided.

## Description

### [Technical Field]

The present disclosure relates to a modification method of a genome sequence, which enables modification of a nucleic acid base in a particular region of a genome, without cleaving double-stranded DNA or inserting a foreign DNA fragment, but utilizing a base excision reaction.

### [Background Art]

In recent years, many studies have been conducted on the production of transformed plants by introducing genes into plants, and successful examples have been reported in many plant species. As such a breeding method for plants, a conventional method including introducing random mutations into the target plant and selecting those that have acquired the desired trait is often adopted. In some cases, transformed plants are produced by introducing mutations in a limited pattern into a limited site by using a genome editing technique that accompanies general DNA double strand cleavage.

### [Summary of Invention]

### [Solution to Problem]

The present inventors have already reported that they have successfully modified, without DNA double strand break, a genome sequence by nucleic acid base conversion in a region containing a particular DNA sequence, by using DNA glycosylase and linking the enzyme and a molecule having a DNA sequence recognition ability (WO 2016/072399). Therefore, the present inventors have further developed this technique, and provide a method for site-specific modification of DNA molecules that can introduce various mutations into not only microorganisms but also higher multicellular organisms (cell populations) including plants and can be finally isolated as individual mutant clones.

Therefore, the present disclosure provides the following.

### (item 1)

A method for producing a plant cell modified at a targeted site of a double-stranded DNA, comprising
(i) a step of providing a plant cell comprising the double-stranded DNA of interest,
(ii) a step of providing a complex in which a nucleic acid sequence-recognizing module that specifically binds to a target nucleotide sequence in the double-stranded DNA and a DNA glycosylase with sufficiently low reactivity with the double-stranded DNA are bound,
(iii) a step of placing the complex in a condition under which the plant cell is transfected,
(iv) a step of placing the transfected plant cell in a condition that induces modification of the targeted site, without cleaving at least one strand of the double-stranded DNA in the targeted site, and
(v) a step of selecting a cell into which the complex has been introduced and/or a cell into which the modification has been introduced.

### (item 2)

The method of the above-mentioned item, wherein the aforementioned nucleic acid sequence-recognizing module is selected from the group consisting of a CRISPR-Cas system wherein at least one DNA cleavage ability of Cas is inactivated, a zinc finger motif, a TAL effector and a PPR motif.

### (item 3)

The method of any one of the above-mentioned items, wherein the aforementioned nucleic acid sequence-recognizing module is a CRISPR-Cas system in which Cas does not have at least one DNA cleavage ability.

### (item 4)

The method of any one of the above-mentioned items, wherein the aforementioned nucleic acid sequence-recognizing module is a CRISPR-Cas system in which Cas does not have both of DNA cleavage ability.

### (item 5)

The method of any one of the above-mentioned items, wherein the aforementioned modification comprises substitution or deletion of one or more nucleotides in the aforementioned targeted site, or insertion of one or more nucleotides in the aforementioned targeted site.

### (item 6)

The method of any one of the above-mentioned items, wherein the aforementioned modification dominantly occurs on the PAM sequence side of the aforementioned targeted site.

### (item 7)

The method of any one of the above-mentioned items, wherein the aforementioned DNA glycosylase is a mutant whose reactivity with double-stranded DNA is attenuated as compared with the wild type.

### (item 8)

The method of any one of the above-mentioned items, wherein the aforementioned DNA glycosylase has cytosine-DNA glycosylase (CDG) activity or thymine-DNA glycosylase (TDG) activity.

### (item 9)

The method of any one of the above-mentioned items, wherein the aforementioned DNA glycosylase having CDG activity or TDG activity is a mutant of uracil-DNA glycosylase (UDG).

### (item 10)

The method of any one of the above-mentioned items, wherein the aforementioned DNA glycosylase is a mutant of uracil-DNA glycosylase (UDG) derived from a yeast and having CDG activity or TDG activity.

### (item 11)

The method of any one of the above-mentioned items, wherein the aforementioned plant cell is derived from rice, Arabidopsis thaliana, bean, maize, cotton, safflower, sunflower, tobacco, wheat, barley, hemp, rose, Japanese yew, banana, coffee, sesame, buckwheat, or lettuce.

### (item 12)

The method of any one of the above-mentioned items, wherein the aforementioned plant cell is derived from rice or Arabidopsis thaliana.

### (item 13)

The method of any one of the above-mentioned items, wherein the aforementioned transfection is performed through delivery of the aforementioned complex to separated plant callus or by the Floral dip method.

### (item 14)

The method of any one of the above-mentioned items, wherein the aforementioned delivery is performed by the Agrobacterium method.

### (item 15)

The method of any one of the above-mentioned items, further comprising a step of producing a plant body from the aforementioned cell.

### (item 16)

The method of any one of the above-mentioned items, further comprising a step of clonally separating the aforementioned obtained cell.

### (item 17)

A transformed plant cell obtainable by the method according to any one of the above-mentioned items.

### (item 18)

A transformed plant comprising the plant cell of any one of the above-mentioned items.

### (item 19)

A seed obtained from the plant of any one of the above-mentioned items.

### (item 20)

The plant of any one of the above-mentioned items, wherein the aforementioned transformed trait is expressed only in the primary transgenic generation.

### (item 21)

The plant of any one of the above-mentioned items, wherein the aforementioned expression of the transformed trait is inherited across generations.

### (item A1)

A method for producing a cell modified at a targeted site of a double-stranded DNA, comprising
(i) a step of providing a cell comprising the double-stranded DNA of interest,
(ii) a step of providing a complex in which a nucleic acid sequence-recognizing module that specifically binds to a target nucleotide sequence in the double-stranded DNA and a DNA glycosylase with sufficiently low reactivity with the double-stranded DNA are bound,
(iii) a step of placing the complex in a condition under which the cell is transfected,
(iv) a step of placing the transfected cell in a condition that induces modification of the targeted site, without cleaving at least one strand of the double-stranded DNA in the targeted site, and
(v) a step of selecting a cell into which the complex has been introduced and/or a cell into which the modification has been introduced, wherein
the modification in the cell is maintained for at least the primary transformant.

### (item A2)

The method of the above-mentioned items, wherein the aforementioned nucleic acid sequence-recognizing module is selected from the group consisting of a CRISPR-Cas system wherein at least one DNA cleavage ability of Cas is inactivated, a zinc finger motif, a TAL effector and a PPR motif.

### (item A3)

The method of any one of the above-mentioned items, wherein the aforementioned nucleic acid sequence-recognizing module is a CRISPR-Cas system in which Cas does not have at least one DNA cleavage ability.

### (item A4)

The method of any one of the above-mentioned items, wherein the aforementioned nucleic acid sequence-recognizing module is a CRISPR-Cas system in which Cas does not have DNA both of DNA cleavage ability.

### (item A5)

The method of any one of the above-mentioned items, wherein the aforementioned modification comprises substitution or deletion of one or more nucleotides in the aforementioned targeted site, or insertion of one or more nucleotides in the aforementioned targeted site.

### (item A6)

The method of any one of the above-mentioned items, wherein the aforementioned modification dominantly occurs on the PAM sequence side of the aforementioned targeted site.

### (item A7)

The method of any one of the above-mentioned items, wherein the aforementioned DNA glycosylase is a mutant whose reactivity with double-stranded DNA is attenuated as compared with the wild type.

### (item A8)

The method of any one of the above-mentioned items, wherein the aforementioned DNA glycosylase has cytosine-DNA glycosylase (CDG) activity or thymine-DNA glycosylase (TDG) activity.

### (item A9)

The method of any one of the above-mentioned items, wherein the aforementioned DNA glycosylase having CDG activity or TDG activity is a mutant of uracil-DNA glycosylase (UDG).

### (item A10)

The method of any one of the above-mentioned items, wherein the aforementioned DNA glycosylase is a mutant of uracil-DNA glycosylase (UDG) derived from a yeast and having CDG activity or TDG activity.

### (item A11)

The method of any one of the above-mentioned items, wherein the aforementioned plant cell is derived from rice, Arabidopsis thaliana, bean, maize, cotton, safflower, sunflower, tobacco, wheat, barley, hemp, rose, Japanese yew, banana, coffee, sesame, buckwheat, or lettuce.

### (item A12)

The method of any one of the above-mentioned items, wherein the aforementioned plant cell is derived from rice or Arabidopsis thaliana.

### (item A13)

The method of any one of the above-mentioned items, wherein the aforementioned transfection is performed through delivery of the aforementioned complex to separated plant callus or by the Floral dip method.

### (item A14)

The method of any one of the above-mentioned items, wherein the aforementioned delivery is performed by the Agrobacterium method.

### (item A15)

The method of any one of the above-mentioned items, further comprising a step of producing a plant body from the aforementioned cell.

### (item A16)

The method of any one of the above-mentioned items, further comprising a step of clonally separating the obtained aforementioned cell.

### (item A17)

A transformed plant cell obtainable by the method according to any one of the above-mentioned items.

### (item A18)

A transformed plant comprising the plant cell of any one of the above-mentioned items.

### (item A19)

A seed obtained from the plant of any one of the above-mentioned items.

### (item A20)

The plant of any one of the above-mentioned items, wherein the aforementioned transformed trait is expressed only in the primary transgenic generation.

### (item A21)

The plant of any one of the above-mentioned items, wherein the aforementioned expression of the transformed trait is inherited across generations.

### (item B1)

A method for producing a plant cell having a desired property, comprising
(i) a step of providing a plant cell comprising a double-stranded DNA related to the desired property,
(ii) a step of providing a complex in which a nucleic acid sequence-recognizing module that specifically binds to a target nucleotide sequence in the double-stranded DNA and a DNA glycosylase with sufficiently low reactivity with the double-stranded DNA are bound,
(iii) a step of placing the complex in a condition under which the plant cell is transfected,
(iv) a step of placing the transfected plant cell in a condition that induces modification of the targeted site, without cleaving at least one strand of the double-stranded DNA in the targeted site,
(v) a step of selecting a cell into which the complex has been introduced and/or a cell into which the modification has been introduced, and
(vi) a step of selecting the cell having the desired property from the introduced cells.

### (item B2)

The method of the above-mentioned item, wherein the aforementioned nucleic acid sequence-recognizing module is selected from the group consisting of a CRISPR-Cas system wherein at least one DNA cleavage ability of Cas is inactivated, a zinc finger motif, a TAL effector and a PPR motif.

### (item B3)

The method of any one of the above-mentioned items, wherein the aforementioned nucleic acid sequence-recognizing module is a CRISPR-Cas system in which Cas does not have at least one DNA cleavage ability.

### (item B4)

The method of any one of the above-mentioned items, wherein the aforementioned nucleic acid sequence-recognizing module is a CRISPR-Cas system in which Cas does not have both of DNA cleavage ability.

### (item B5)

The method of any one of the above-mentioned items, wherein the aforementioned modification comprises substitution or deletion of one or more nucleotides in the aforementioned targeted site, or insertion of one or more nucleotides in the aforementioned targeted site.

### (item B6)

The method of any one of the above-mentioned items, wherein the aforementioned modification dominantly occurs on the PAM sequence side of the aforementioned targeted site.

### (item B7)

The method of any one of the above-mentioned items, wherein the aforementioned DNA glycosylase is a mutant whose reactivity with double-stranded DNA is attenuated as compared with the wild type.

### (item B8)

The method of any one of the above-mentioned items, wherein the aforementioned DNA glycosylase has cytosine-DNA glycosylase (CDG) activity or thymine-DNA glycosylase (TDG) activity.

### (item B9)

The method of any one of the above-mentioned items, wherein the aforementioned DNA glycosylase having CDG activity or TDG activity is a mutant of uracil-DNA glycosylase (UDG).

### (item B10)

The method of any one of the above-mentioned items, wherein the aforementioned DNA glycosylase is a mutant of uracil-DNA glycosylase (UDG) derived from a yeast and having CDG activity or TDG activity.

### (item 11)

The method of any one of the above-mentioned items, wherein the aforementioned plant cell is derived from rice, Arabidopsis thaliana, bean, maize, cotton, safflower, sunflower, tobacco, wheat, barley, hemp, rose, Japanese yew, banana, coffee, sesame, buckwheat, or lettuce.

### (item B12)

The method of any one of the above-mentioned items, wherein the aforementioned plant cell is derived from rice or Arabidopsis thaliana.

### (item B13)

The method of any one of the above-mentioned items, wherein the aforementioned transfection is performed through delivery of the aforementioned complex to separated plant callus or by the Floral dip method.

### (item B14)

The method of any one of the above-mentioned items, wherein the aforementioned delivery is performed by the Agrobacterium method.

### (item B15)

The method of any one of the above-mentioned items, further comprising a step of producing a plant body from the aforementioned cell.

### (item B16)

The method of any one of the above-mentioned items, further comprising a step of clonally separating a cell having the aforementioned desired property.

### (item B17)

A transformed plant cell obtainable by the method according to any one of the above-mentioned items.

### (item B18)

A transformed plant comprising the plant cell of any one of the above-mentioned items.

### (item B19)

A seed obtained from the plant of any one of the above-mentioned items.

### (item B20)

The plant of any one of the above-mentioned items, wherein the aforementioned transformed trait is expressed only in the primary transgenic generation.

### (item B21)

The plant of any one of the above-mentioned items, wherein the aforementioned expression of the transformed trait is inherited across generations.

### (item C1)

A complex for producing a plant cell modified at a targeted site of a double-stranded DNA, in which a nucleic acid sequence-recognizing module that specifically binds to a target nucleotide sequence in the double-stranded DNA of the plant cell and a DNA glycosylase with sufficiently low reactivity with the double-stranded DNA are bound, wherein modification of the targeted site is induced without cleaving at least one strand of the double-stranded DNA in the targeted site.

### (item C2)

A complex for producing a cell modified at a targeted site of a double-stranded DNA, in which a nucleic acid sequence-recognizing module that specifically binds to a target nucleotide sequence in the double-stranded DNA of the cell and a DNA glycosylase with sufficiently low reactivity with the double-stranded DNA are bound, wherein modification of the targeted site is induced without cleaving at least one strand of the double-stranded DNA in the targeted site.

### (item C3)

A complex for producing a plant cell having a desired property, in which a nucleic acid sequence-recognizing module that specifically binds to a target nucleotide sequence in the double-stranded DNA of the plant cell and a DNA glycosylase with sufficiently low reactivity with the double-stranded DNA are bound, wherein modification of the targeted site is induced without cleaving at least one strand of the double-stranded DNA in the targeted site.

### (item C4)

One or more nucleic acids encoding the complex according to any one of the the above-mentioned items.

### (item C5)

One or more nucleic acids encoding the complex according to any one of the above-mentioned items, which is/are used in combination with other nucleic acid encoding the complex.

The present disclosure is intended so that one or more of the aforementioned features can be provided not only as the explicitly disclosed combinations, but also as other combinations thereof. Additional embodiments and advantages of the present disclosure are recognized by those skilled in the art by reading and understanding the following detailed description as needed.

The features and remarkable actions and effects of the present disclosure other than those described above will become clear to those skilled in the art by referring to the following embodiments of the invention and the drawings.

### [Advantageous Effects of Invention]

It has been heretofore demonstrated that point mutations can be induced around the target site in yeast, but genetic modification has not been possible in higher multicellular organisms (cell populations) including plants. The method of the present disclosure has made it possible to generate not only point mutations but also deletions in not only conventional microorganisms but also plants, which are multicellular organisms, and is expected to be applied as a new genome editing technique.

### [Brief Description of Drawings]

[Fig. 1]

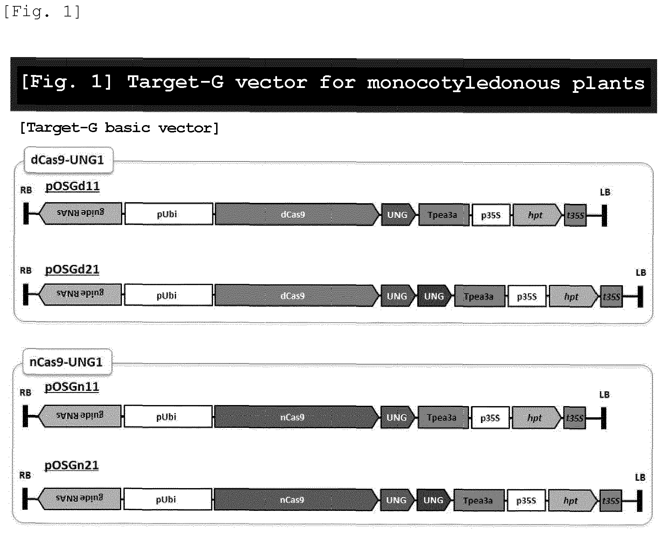

   Fig. 1 is a schematic diagram showing the Target-G vector for monocot in one embodiment of the present disclosure.
[Fig. 2]

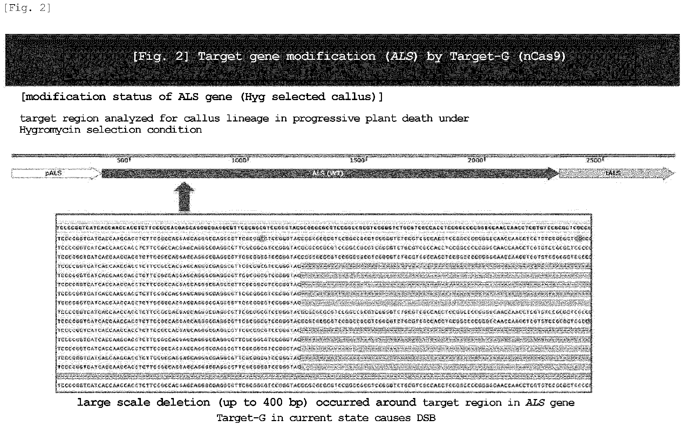

   Fig. 2 is a schematic diagram showing the results of target genetic modification by Target-G (nCas9) when ALS gene is the target in one embodiment of the present disclosure.
[Fig. 3]

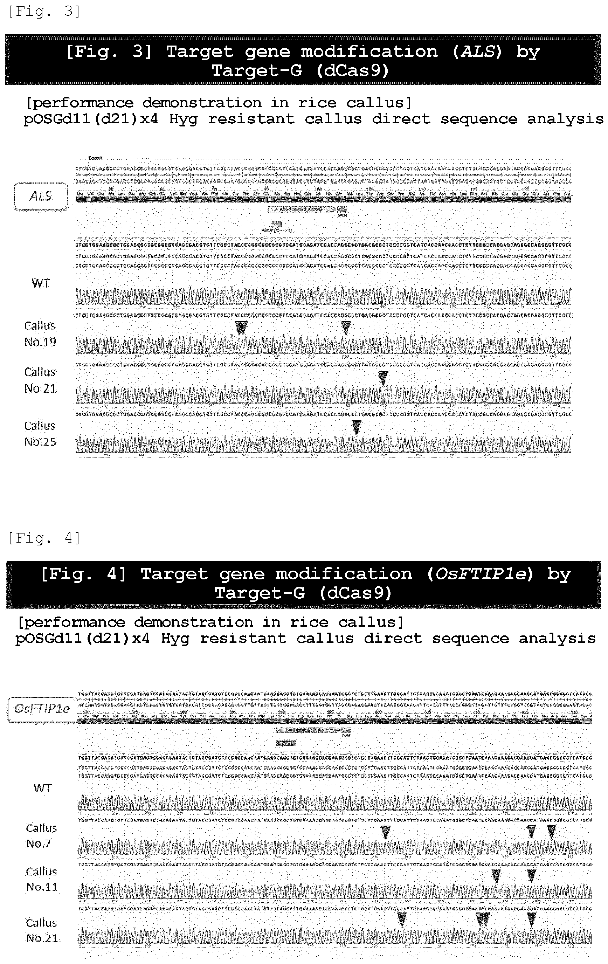

   Fig. 3 is a schematic diagram showing the results of target genetic modification by Target-G (dCas9) when ALS gene is the target in one embodiment of the present disclosure.
[Fig. 4]
   Fig. 4 is a schematic diagram showing the results of target genetic modification by Target-G (dCas9) when OsFTIPle gene is the target in one embodiment of the present disclosure.
[Fig. 5]

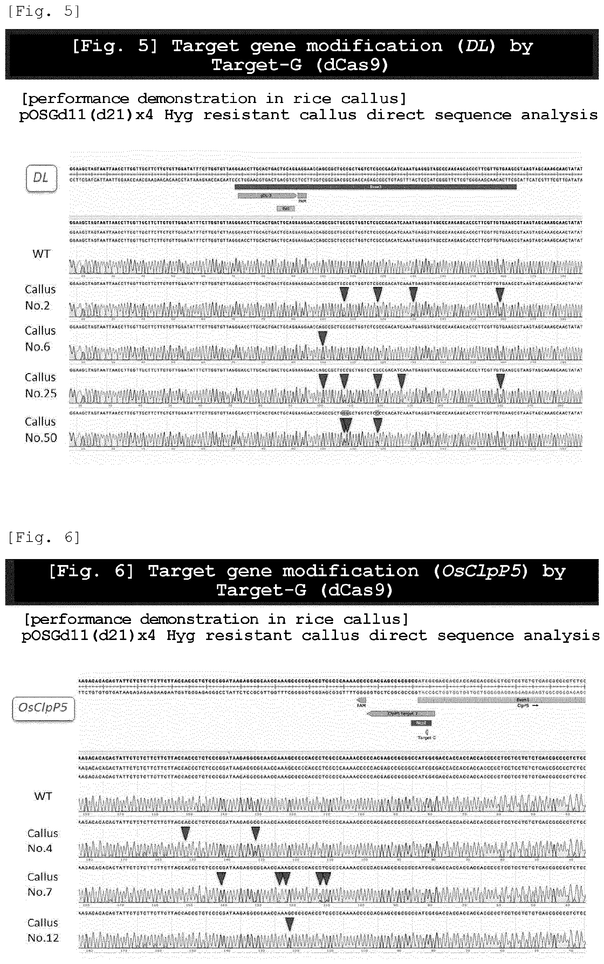

   Fig. 5 is a schematic diagram showing the results of target genetic modification by Target-G (dCas9) when DL gene is the target in one embodiment of the present disclosure.
[Fig. 6]
   Fig. 6 is a schematic diagram showing the results of target genetic modification by Target-G when OsClpP5 gene is the target in one embodiment of the present disclosure.
[Fig. 7]

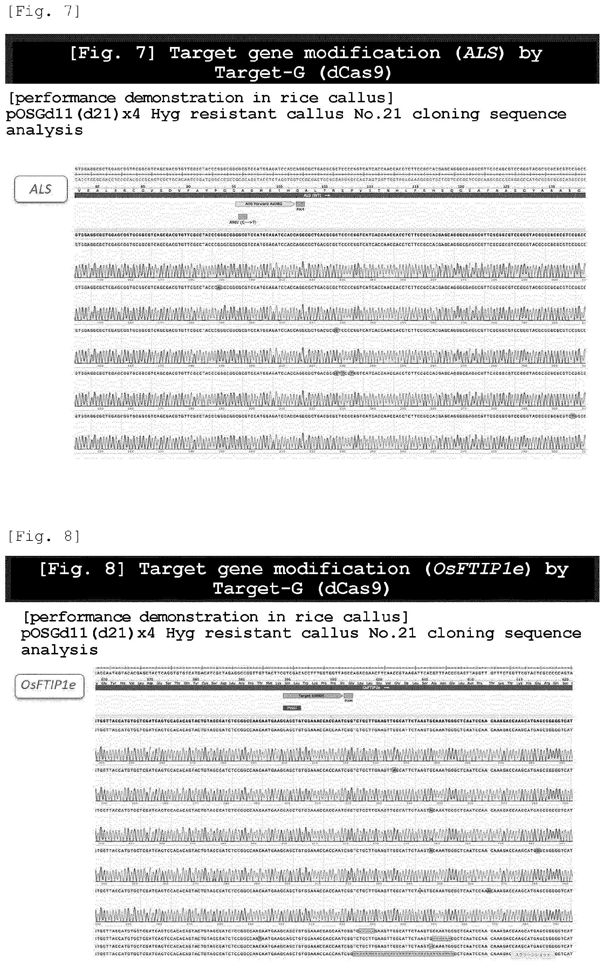

   Fig. 7 is a schematic diagram showing the results of cloning sequence analysis in a callus lineage when ALS gene is the target in one embodiment of the present disclosure.
[Fig. 8]
   Fig. 8 is a schematic diagram showing the results of cloning sequence analysis in a callus lineage when OsFTIPle gene is the target in one embodiment of the present disclosure.
[Fig. 9]

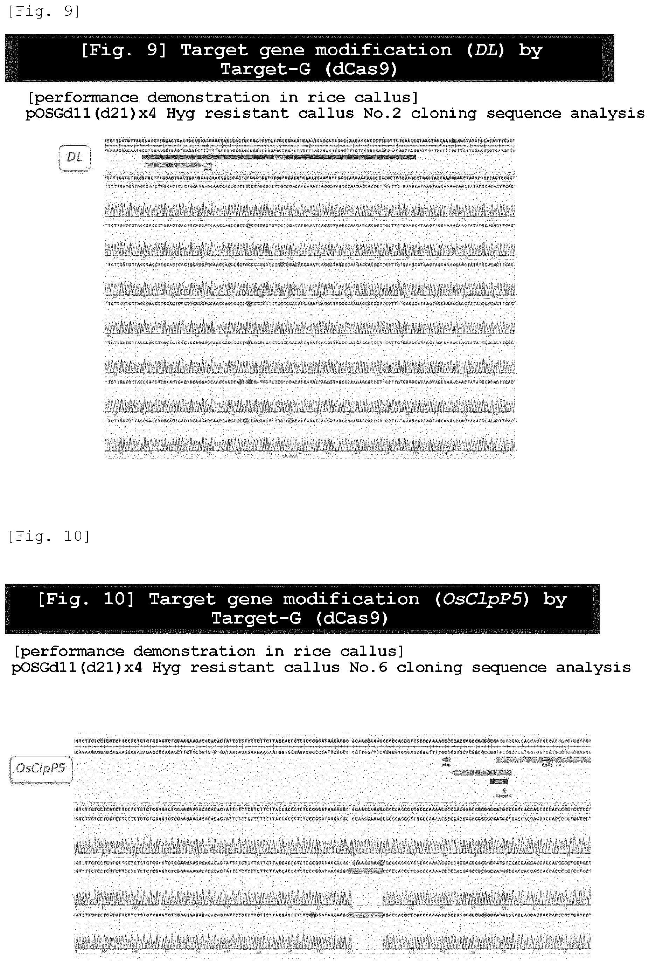

   Fig. 9 is a schematic diagram showing the results of cloning sequence analysis in a callus lineage when DL gene is the target in one embodiment of the present disclosure.
[Fig. 10]
   Fig. 10 is a schematic diagram showing the results of cloning sequence analysis in a callus lineage when OsClpP5 gene is the target in one embodiment of the present disclosure.
[Fig. 11]

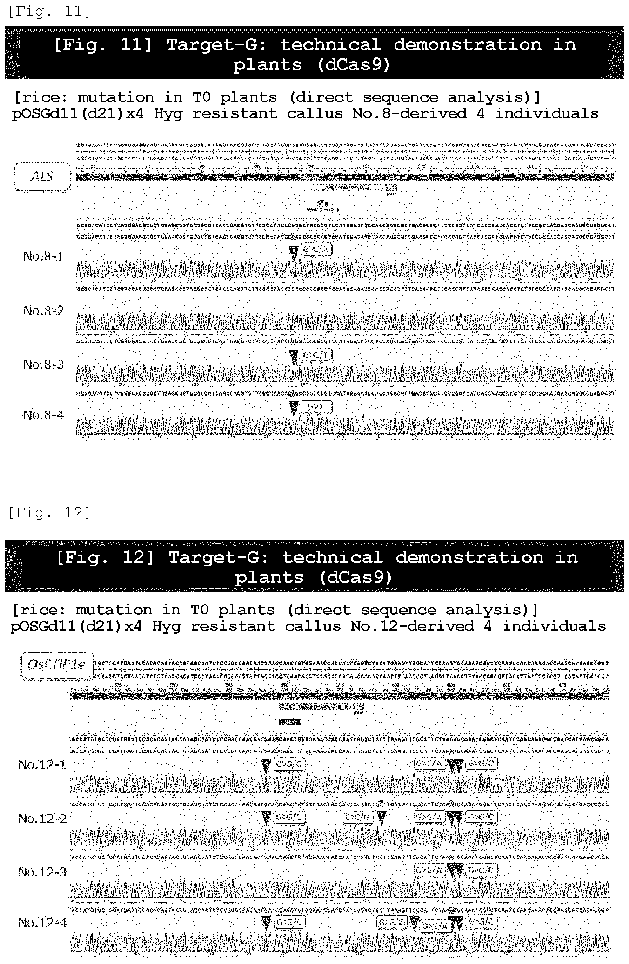

   Fig. 11 is a schematic diagram showing the results of confirmation that mutations confirmed in callus when ALS gene is the target are also transferred to T0 plants in one embodiment of the present disclosure.
[Fig. 12]
   Fig. 12 is a schematic diagram showing the results of confirmation that mutations confirmed in callus when OsFTIPle gene is the target are also transferred to T0 plants in one embodiment of the present disclosure.
[Fig. 13]

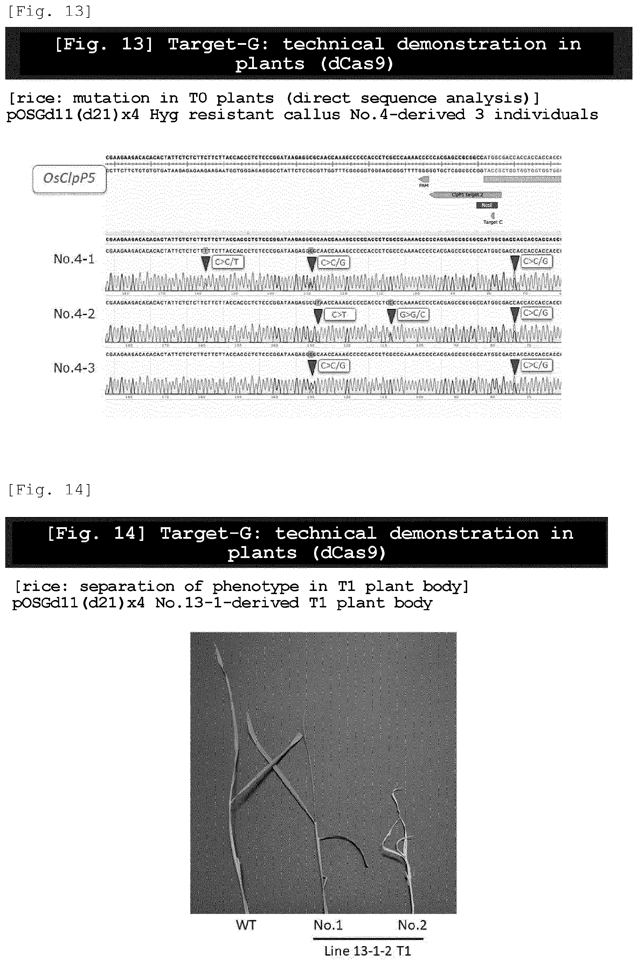

   Fig. 13 is a schematic diagram showing the results of confirmation that mutations confirmed in callus when OsClpP5 gene is the target are also transferred to T0 plants in one embodiment of the present disclosure.
[Fig. 14]
   Fig. 14 is a photograph showing that mutations in T0 plants whose genes have been modified by the method of the present disclosure are also transferred to T1 generation plants in one embodiment of the present disclosure.

### [Description of Embodiments]

The present disclosure is described hereinafter while showing the best modes thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

The definitions of the terms and/or basic techniques particularly used in the present specification are described as appropriate in the following.

In the present specification, "about" means ±10% of the numerical value that follows.

In the present specification, the "modification" of a double-stranded DNA means that a nucleotide (e.g., dC) on a DNA strand is converted to other nucleotide (e.g., dT, dA or dG), or deleted, or a nucleotide or a nucleotide sequence is inserted between certain nucleotides on a DNA strand. The "modification" in the present specification includes substitution or deletion of one or more nucleotides in the targeted site of a double-stranded DNA, or insertion of one or more nucleotides in the targeted site of a double-stranded DNA. While the double-stranded DNA to be modified is not particularly limited, it is preferably a genomic DNA. The "targeted site" of a double-stranded DNA means the whole or partial "target nucleotide sequence", which a nucleic acid sequence-recognizing module specifically recognizes and binds to, or the vicinity of the target nucleotide sequence (one or both of 5' upstream and 3' downstream), and the range thereof can be appropriately adjusted between 1 base and several hundred bases according to the object.

In the present specification, the "nucleic acid sequence-recognizing module" means a molecule or molecule complex having an ability to specifically recognize and bind to a particular nucleotide sequence (i.e., target nucleotide sequence) on a DNA strand. Binding of the nucleic acid sequence-recognizing module to a target nucleotide sequence enables a DNA glycosylase linked to the module to specifically act on a targeted site of a double-stranded DNA.

In the present specification, "DNA glycosylase" means an enzyme that hydrolyzes N-glycosidic bond of DNA. DNA glycosylase originally plays a role of eliminating injured bases from DNA in the base excision repair (BER) mechanism. In the present disclosure, DNA glycosylase capable of acting on normal bases (i.e., dC, dT, dA and dG, or those after epigenetic modification) in DNA is preferable. A mutant DNA glycosylase which natively does not react with a normal base or has low reactivity but which acquired reactivity or has improved reactivity with a normal base by mutation is encompassed in the DNA glycosylase in the present invention and can be preferably used. An abasic site (apurinic/apyrimidic (AP) site) resulting from the base excision reaction by the enzyme is treated with an enzyme at the downstream of the BER pathway, such as AP endonuclease, DNA polymerase, DNA ligase and the like.

In the present specification, "sufficiently low reactivity with a double-stranded DNA" means that a base excision reaction in a region where a double-stranded DNA is formed is performed only at a frequency sufficient to suppress cytotoxicity to a level uninfluential on the survival of the cells. Examples of the DNA glycosylase with sufficiently low reactivity with a double-stranded DNA include DNA glycosylase with natively sufficiently low reactivity with a double-stranded DNA, mutant DNA glycosylase introduced with a mutation to decrease reactivity with a double-stranded DNA as compared to wild-type and the like. Furthermore, DNA glycosylase which is a split enzyme designed to be split into two fragments, wherein respective fragments are bonded to either one of the nucleic acid sequence-recognizing module split into two to form two complexes, when the both complexes are refolded, the nucleic acid sequence-recognizing module can be specifically bonded to the target nucleotide sequence, and, due to the specific bond, the DNA glycosylase can catalyze the base excision reaction is also encompassed in the "DNA glycosylase with sufficiently low reactivity with a double-stranded DNA" in the present specification.

In the present specification, the above-mentioned "complex" in which a nucleic acid sequence-recognizing module and a DNA glycosylase are bound means a molecule complex comprising a complex wherein the above-mentioned nucleic acid sequence-recognizing module and a DNA glycosylase with sufficiently low reactivity with a double-stranded DNA are linked, which molecule complex is imparted with a particular nucleotide sequence recognition ability, and an activity to catalyze a base excision reaction of nucleic acid. The "complex" here encompasses not only one constituted of multiple molecules, but also one having a nucleic acid sequence-recognizing module and DNA glycosylase in a single molecule, like a fusion protein. In addition, the "complex" in the present invention also encompasses a molecule complex in which nucleic acid sequence-recognizing module split into two fragments and either fragments of DNA glycosylase split into two fragments are linked to each other to form two "partial complexes", and the molecule complex acquires a nucleotide sequence recognition ability and a base excision reaction catalyst activity when the partial complexes are refolded with each other.

In the present specification, "transfection" refers to the introduction of a molecule, such as a nucleic acid or the like, into a cell or host organism and is used interchangeably with "transduction" and "transformation". Mutations resulting from the introduction of nucleic acids or the above-mentioned complex may occur in the genomic nucleic acid of a recipient cell. When the mutation occurs in the nucleic acid (genomic DNA) of the recipient cell or organism, the mutation can be stably maintained in that cell or organism, further passed on to the recipient cell, or progeny of the organism, or an organism, or inherited by them to the next generation.

In the present specification, the "transfected plant cell" or "transformed plant cell" refers to a plant cell into which a molecule of a nucleic acid or the like or the above-mentioned complex has been transfected. The "transfected plant cell" or "transformed plant cell" includes a plant cell whose genetic traits have been altered by the expression of a foreign gene integrated into the plant genome, or a plant cell in which a foreign gene has been simply introduced into a leaf, stem, or root. The "transformed plant" refers to a plant individual consisting of such cells.

In the present specification, the "cell into which a complex has been introduced" or "cell into which a modification has been introduced" refers to a cell into which the above-mentioned complex has been introduced as a result of transfection of the cell as described above, or a cell in which substitution or deletion of one or more nucleotides or insertion of one or more nucleotides in the targeted site of a double-stranded DNA have occurred by the action of the complex.

Since the transformed plant of the present disclosure is a plant individual, it has reproductive capacity (ability to reproduce sexually), and has a superior advantage that the mutation introduced into the plant genome and traits thereof are also inherited to the next generation.

### (Preferred embodiment)

Preferred embodiments of the present disclosure are described below. The embodiments provided below are provided for a better understanding of the disclosure, and it is understood that the scope of the disclosure should not be limited to the following description. Therefore, it is clear that a person skilled in the art can make appropriate modifications within the scope of the present disclosure in light of the description in the present specification. It is also understood that the following embodiments of the disclosure can be used singly or in combination.

In one aspect of the present disclosure, a method for producing a plant cell modified at a targeted site of a double-stranded DNA, including (i) a step of providing a plant cell comprising the double-stranded DNA of interest, (ii) a step of providing a complex in which a nucleic acid sequence-recognizing module that specifically binds to a target nucleotide sequence in the double-stranded DNA and a DNA glycosylase with sufficiently low reactivity with the double-stranded DNA are bound, (iii) a step of placing the complex in a condition under which the plant cell is transfected, (iv) a step of placing the transfected plant cell in a condition that induces modification of the targeted site, without cleaving at least one strand of the double-stranded DNA in the targeted site, and (v) a step of selecting a cell into which the complex has been introduced and/or a cell into which the modification has been introduced is provided. The method of the present disclosure is useful as a genome editing technique for multicellular organisms and as a subsequent isolation technique for mutant clones.

In one aspect of the present disclosure, a method for producing a cell modified at a targeted site of a double-stranded DNA, including (i) a step of providing a cell comprising the double-stranded DNA of interest, (ii) a step of providing a complex in which a nucleic acid sequence-recognizing module that specifically binds to a target nucleotide sequence in the double-stranded DNA and a DNA glycosylase with sufficiently low reactivity with the double-stranded DNA are bound, (iii) a step of placing the complex in a condition under which the cell is transfected, (iv) a step of placing the transfected cell in a condition that induces modification of the targeted site, without cleaving at least one strand of the double-stranded DNA in the targeted site, and (v) a step of selecting a cell into which the complex has been introduced and/or a cell into which the modification has been introduced, wherein the modification in the cell is maintained for at least the primary transformant, is provided.

In one aspect of the present disclosure, a method for producing a plant cell having a desired property, including (i) a step of providing a plant cell comprising a double-stranded DNA related to the desired property, (ii) a step of providing a complex in which a nucleic acid sequence-recognizing module that specifically binds to a target nucleotide sequence in the double-stranded DNA and a DNA glycosylase with sufficiently low reactivity with the double-stranded DNA are bound, (iii) a step of placing the complex in a condition under which the plant cell is transfected, (iv) a step of placing the transfected plant cell in a condition that induces modification of the targeted site, without cleaving at least one strand of the double-stranded DNA in the targeted site, (v) a step of selecting a cell into which the complex has been introduced and/or a cell into which the modification has been introduced, and (vi) a step of selecting the cell having the desired property from the introduced cells is provided.

When obtaining transformants in multicellular organisms such as plant and the like, conventional introduction of random mutations and selection of those that have acquired desired traits is very inefficient. General genome editing techniques that generate cleavage of double strands can only introduce mutations in limited patterns at limited sites. Therefore, the present disclosure provides a novel genome editing technique capable of introducing various mutations into a wide range of target regions in target genomes such as plant genomes. Such techniques have so far been realized in microorganisms, but have technical difficulties in higher multicellular organisms.

While DNA glycosylase to be used in the present disclosure is not particularly limited as long as it can hydrolyze N-glycosidic bond of DNA to catalyze a reaction for releasing a base, DNA glycosylase capable of acting on normal bases (i.e., dC, dT, dA and dG, or those after epigenetic modification, for example, 5-methylcytosine etc.) in DNA is preferable to increase versatility as a genome editing technique. Examples of such enzyme include an enzyme having CDG activity to catalyze a reaction for releasing cytosine, an enzyme having TDG activity to catalyze a reaction for releasing thymine, an enzyme having activity to catalyze a reaction for releasing 5-methylcytosine (5-mCDG activity) and the like. While DNA glycosylase natively having CDG activity has not been reported to date, in almost all species, a mutant UDG having CDG activity or TDG activity that recognizes cytidine or thymidine as a substrate can be obtained by introducing a mutation into a particular amino acid residue of UNG known as the major UDG responsible for the removal of uracil incorporated in DNA (in the case of eucaryote, it has two splice variants of mitochondrial localization UNG1 and nuclear localization UNG2, which have a common amino acid sequence except N-terminal sequence containing each oraganelle localization signal). More specifically, for example, in the case of UNG1 derived from yeast (GenBank accession No. NP_013691), CDG activity can be imparted by substituting the 222nd asparagine from the N-terminal with aspartic acid (the mutant is also referred to as N222D), and TDG activity can be imparted by substituting the 164th tyrosine with alanine (the mutant is also referred to as Y164A) (Kavli B. et al., EMBO J. (1996) 15(13): 3442-7). The present inventors have found that TDG activity higher than that of Y164A mutant can be imparted by substituting the 164th tyrosine with glycine (the mutant is also referred to as Y164G). Furthermore, since cytosine is obtained by substituting the carbonyl group at the 4-position of uracil with an amino group and thymine is equivalent to uracil in which the 5-position is methylated, DNA glycosylase having an activity to release 5-methylcytosine, derived from methylation of the 5-position of cytosine, from DNA (5-mCDG activity) can be obtained by introducing double mutation into the 222nd asparagine residue and the 164th tyrosine residue (e.g., N222D/Y164A or N222D/Y164G). Since base excision of 5-methylcytosine can change the methylation state of the genomic DNA, the genome editing technique of the present disclosure also enables epigenome editing to change epigenetic modification.

When UNG2 is used in one embodiment of the present disclosure, mutant UNG having CDG activity, TDG activity or 5-mCDG activity can be obtained by introducing similar mutation into the amino acid residue corresponding to the above-mentioned mutated site.

In the mutant UNG in one embodiment of the present disclosure, the above-mentioned site may be substituted with an amino acid other than the above-mentioned amino acid, or the mutation may be introduced into a site other than the above-mentioned site, as long as it can act on a normal base. Whether the mutant UNG can act on a normal base can be confirmed by the method described in, for example, Kavli B. et al., EMBO J. (1996) 15(13): 3442-7.

In one embodiment of the present disclosure, while the derivation of UNG is not particularly limited, for example, ung derived Escherichia coli (Varshney, U. et al. (1988) J. Biol. Chem., 263, 7776-7784), UNG1 or UNG2 derived from yeast, mammal (e.g., human, mouse, swine, bovine, horse, monkey etc.) and the like, or UDG derived from virus (e.g., Poxviridae (vaccinia virus etc.), Herpesviridae and the like) can be used. For example, UniprotKB Nos. P13051-2 and P13051-1 can be referred to for the amino acid sequences of human UNG1 and UNG2, respectively. In addition, UniprotKB No. P12295 can be referred to for the amino acid sequence of Escherichia coli (K-12 strain) ung, and UniprotKB No. P20536 can be referred to for the amino acid sequence of vaccinia virus (Copenhagen Strain) UDG. The amino acid sequence of UNG is highly preserved among species, and the corresponding site for mutation can be identified by aligning the amino acid sequence of UNG1 or UNG2 derived from a desired organism with that of the above-mentioned yeast UNG1. For example, in the case of human UNG1, the amino acid corresponding to N222 of yeast UNG1 is the 204th asparagine (N204), and the amino acid corresponding to Y164 is the 147th tyrosine (Y147). In the case of Escherichia coli ung, the amino acid corresponding to N222 of yeast UNG1 is the 123rd asparagine (N123), and the amino acid corresponding to Y164 is the 66th tyrosine (Y66). In the case of UDG derived from Poxviridae virus such as vaccinia virus, smallpoxvirus, monkeypoxvirus, fowlpox virus, swinepox virus, rabbit fibroma virus, the amino acid corresponding to N222 of yeast UNG1 is the 120th asparagine (N120), and the amino acid corresponding to Y164 is the 70th tyrosine (Y70).

While UNG can remove uracil from single stranded DNA and double-stranded DNA, it has higher affinity for single stranded DNA. This tendency is also found in the above-mentioned mutant UNG conferred with CDG activity or TDG activity. However, since cytosine and thymine exist everywhere in the genomic DNA, mutant UNG having CDG activity or TDG activity acts, unlike wild-type UNG that exclusively uses, as a substrate, uracil which is rarely introduced into genomic DNA by an error on replication or deamination of cytosine, on any site in the nucleotide sequence targeted by a nucleic acid sequence-recognizing module and double-stranded DNA in the vicinity thereof to remove cytosine or thymine, thus causing strong cytotoxicity. Therefore, DNA glycosylase to be used in the present disclosure is required to show sufficiently low reactivity with a double-stranded DNA. When mutant UNG is used as DNA glycosylase, the cytotoxicity by the enzyme can be avoided by further introducing a mutation that decreases reactivity with a double-stranded DNA to make a base excision reaction by mutant UNG having CDG activity or TDG activity more selective to the region of a relaxed double-stranded or single stranded DNA. Specifically, for example, in the case of UNG, a mutation that decreases the reactivity with U-A 25 mer double-stranded DNA to not more than 1/20, more preferably not more than 1/50, further preferably not more than 1/100, in vitro, that of the wild-type can be mentioned. However, as long as the reactivity with a double-stranded DNA is decreased to a level free from lethal cytotoxicity in vivo, it is not limited by the reactivity in vitro. Whether the DNA glycosylase to be used has "sufficiently low reactivity with a double-stranded DNA" can be confirmed, for example, as disclosed in WO 2016/072399, by inserting the DNA glycosylase into the construct, introducing the construct together with the guide RNA into the object cell, culturing the obtained transformant, and verifying the survivability thereof. Alternatively, a candidate of DNA glycosylase with "sufficiently low reactivity with a double-stranded DNA" can also be screened for by evaluating, as one of the criteria, the reactivity with a double-stranded DNA oligomer in vitro by the method described in Chen, C.Y. et al. DNA Repair (Amst) (2005) 4(7): 793-805. In one embodiment, the "double strand DNA" can mean DNA that has formed a strong double helix structure. For example, the "double-stranded DNA" does not include not only the state of single-stranded DNA in which the bases to form a pair are completely dissociated, but also the state of loose double strands in which base pairs are formed but double helical structure has unwound (relaxed double-stranded DNA).

As a DNA glycosylase fulfilling the above-mentioned conditions in the case of, for example, UNG1 derived from yeast, a mutant in which the 304th leucine from the N-terminal is substituted with alanine can be mentioned (the mutant is also referred to as L304A) (Slupphaug, G. et al. Nature (1996) 384(6604): 87-92). Alternatively, a mutant in which the 308th arginine is substituted with glutamic acid or cysteine (the mutants are also referred to as R308E, R308C, respectively) also shows remarkably decreased reactivity with a double-stranded DNA (Chen, C.Y. et al. DNA Repair (Amst) (2005) 4(7): 793-805). In another embodiment, when a UNG derived from a different species is used, the corresponding site for mutation can be identified by aligning the amino acid sequence of UNG1 or UNG2 derived from a desired organism with that of the above-mentioned yeast UNG1. For example, in the case of human UNG1, the amino acid corresponding to L304 of yeast UNG1 is the 272nd leucine (L272), and the amino acid corresponding to R308 is the 276th arginine (R276). In the case of Escherichia coli ung, the amino acid corresponding to L304 of yeast UNG1 is the 191st leucine (L191), and the amino acid corresponding to R308 is the 195th arginine (R195). In the case of vaccinia virus UDG, the amino acid corresponding to R308 of yeast UNG1 is the 187th arginine (R187).

The substrate specificity and the site of mutation that changes the reactivity with a double-stranded DNA of UNG1 (ung) from yeast (Saccharomyces cerevisiae), Escherichia coli, human or Poxviridae virus, are shown in Table 1. Mutant amino acid is indicated with one letter, and the number shows the position of mutant amino acid when N-terminal amino acid is 1.

**Table 1**

| yeast | Escherichia coli | human | pox virus | change of mutant form and phenotype |
|---|---|---|---|---|
| Y164 | Y66 | Y147 | Y70 | recognizes thymine by mutation to A, G |
| N222 | N123 | N204 | N120 | recognizes cytosine by mutation to D |
| L304 | L191 | L272 | - | reactivity with a double-stranded DNA decreases by mutation to A |
| R308 | R195 | R276 | R187^{a)} | reactivity with a double-stranded DNA decreases by mutation to E, C |

| | | | | |
|---|---|---|---|---|
| a) In vaccinia virus UDG etc., wild-type itself is considered to have natively low reactivity with a double-stranded DNA . | | | | |

From the above, in one embodiment of the present disclosure, preferable examples of the "DNA glycosylase with sufficiently low reactivity with a double-stranded DNA" to be used in the present disclosure include N222D/L304A double mutant, N222D/R308E double mutant, N222D/R308C double mutant, Y164A/L304A double mutant, Y164A/R308E double mutant, Y164A/R308C double mutant, Y164G/L304A double mutant, Y164G/R308E double mutant, Y164G/R308C double mutant, N222D/Y164A/L304A triple mutant, N222D/Y164A/R308E triple mutant, N222D/Y164A/R308C triple mutant, N222D/Y164G/L304A triple mutant, N222D/Y164G/R308E triple mutant, N222D/Y164G/R308C triple mutant and the like of yeast UNG1. In another embodiment, when different UNG is used instead of yeast UNG1, a mutant having an amino acid corresponding to each of the above-mentioned mutants, which is introduced with a similar mutation, may be used.

Alternatively, in another embodiment, as a "DNA glycosylase with sufficiently low reactivity with a double-stranded DNA", DNA glycosylase with natively low reactivity with a double-stranded DNA, and high selectivity to relaxed double-stranded or single stranded DNA can also be used. Examples of such DNA glycosylase include SMUG1 having UDG activity (Single strand-selective Monofunctional Uracil-DNA Glycosylase). While a mutation conferring CDG activity or TDG activity to SMUG1 is not known, it has been reported that the SMUG1 is important for removal of uracil resulting from deamination of cytosine (Nilsen, H. et al. EMBO J. (2001) 20: 4278-4286). The present inventors have already developed a method for specifically introducing a mutation into the targeted nucleotide sequence and the vicinity thereof, by combining cytidine deaminase capable of converting cytosine to uracil, and a nucleic acid sequence-recognizing module similar to that in the present disclosure (WO 2015/133554). By combining with the technique, cytosine can be artificially converted to uracil in the targeted site in genomic DNA, after which SMUG1 can be further reacted to release the uracil from DNA. While the derivation of SMUG1 is not particularly limited, for example, SMUG1 derived from Escherichia coli, yeast, mammal (e.g., human, mouse, swine, bovine, horse, monkey etc.) and the like can be used. For example, UniprotKB No. Q53HC7-1 and Q53HV7-2 can be referred to for the amino acid sequences of the two isoforms of human SMUG1. In addition, while the derivation of cytidine deaminase is not particularly limited, for example, Petromyzon marinus-derived PmCDA1 (Petromyzon marinus cytosine deaminase 1), or AID (Activation-induced cytidine deaminase; AICDA) derived from mammal (e.g., human, swine, bovine, horse, monkey etc.) can be used. For example, GenBank accession No. EF094822 and ABO15149 can be referred to for the base sequence of cDNA and the amino acid sequence of PmCDA1, and GenBank accession No. NM_020661 and NP_065712 can be referred to for the base sequence of cDNA and the amino acid sequence of human AID.

In another embodiment, as the DNA glycosylase with natively low reactivity with a double-stranded DNA, UDG derived from virus belonging to Poxviridae such as vaccinia virus and the like can be mentioned. As shown in WO 2016/072399, vaccinia virus-derived UDG (vvUDG) is considered to be sufficiently low in the reactivity with a double-stranded DNA to a level free from toxicity in a host cell, because it shows growth equivalent to that in yeast UNG1 and Escherichia coli ung, introduced with a mutation that decreases reactivity with a double-stranded DNA (e.g., R187C), on a nonselective medium, even when such mutation is not introduced. When UDG derived from Poxviridae virus such as vvUDG and the like is used as a DNA glycosylase, a mutation (e.g., R187C) corresponding to the mutation that decreases reactivity with a double-stranded DNA in yeast UNG1 can be further introduced. However, when such mutation decreases the CDG activity (N120D) and TDG activity (Y70A, Y70G) of UDG, it is desirably avoided. From the above, preferable examples of UDG derived from Poxviridae virus such as vvUDG to be used in the present disclosure include N120D mutant, Y70G or Y70A mutant, N120D/Y70G double mutant or N120D/Y70A double mutant and the like.

In one embodiment of the present disclosure, when UDG derived from Poxviridae virus such as mutant vvUDG and the like is used as the DNA glycosylase, it is preferable to contact A20 protein, which interacts with UDG to form a heterodimer that functions as a processivity factor of viral DNA polymerase, with double-stranded DNA together with UDG. Since combined use with A20 protein increases CDG activity or TDG activity of mutant UDG, for example, the efficiency of mutation induction into thymine, of mutant vvUDG having low TDG activity (Y70G) as compared to CDG activity (N120D) can be improved by the combined use with A20 protein. While the derivation of A20 is not particularly limited, for example, A20 derived from virus belonging to Poxviridae such as vaccinia virus, smallpoxvirus, monkeypoxvirus, fowlpox virus, swinepox virus, rabbit fibroma virus can be used. For example, UniprotKB No. P20995 can be referred to for the amino acid sequence of vaccinia virus (Copenhagen Strain) A20.

In yet another embodiment, a split enzyme designed such that nucleic acid sequence-recognizing module and DNA glycosylase are each split into two fragments, either fragments are linked to each other to form two partial complexes, these complexes are associated to reconstitute a functional nucleic acid sequence-recognizing module, and the module is bonded to the target nucleotide sequence to reconstitute a functional DNA glycosylase can be used as a DNA glycosylase having low reactivity with a double-stranded DNA. In the split enzyme, since the enzyme activity is exhibited only when it is bonded to the target nucleotide sequence, even when the DNA glycosylase itself to be reconstituted does not have a reduced reactivity with a double-stranded DNA, it consequently acts selectively on the single stranded DNA or relaxed double-stranded DNA part in the target nucleotide sequence and the vicinity thereof. For example, nucleic acid sequence-recognizing module and DNA glycosylase are each split into N-terminal side fragments and C-terminal side fragments, for example, N-terminal side fragments are linked to each other to give a partial complex, C-terminal side fragments are linked to each other to give a partial complex (or N-terminal side fragments of nucleic acid sequence-recognizing module and C-terminal side fragments of DNA glycosylase are linked to give a partial complex, and N-terminal side fragments of DNA glycosylase and C-terminal side fragments of nucleic acid sequence-recognizing module are linked to give a partial complex), and they are associated, whereby functional nucleic acid sequence-recognizing module and functional DNA glycosylase can be reconstituted. The combination of the fragments to be linked is not particularly limited as long as a complex of the functional nucleic acid sequence-recognizing module and the functional DNA glycosylase is reconstituted when the two partial complexes are associated. The two partial complexes may be provided as separate molecules, or may be provided as a single fusion protein by linking them directly or via a suitable linker. The split site in DNA glycosylase is not particularly limited as long as two split fragments can be reconstituted as functional DNA glycosylase, and DNA glycosylase may be split at one site to provide N-terminal side fragment and C-terminal side fragment, or not less than 3 fragments obtained by splitting at two or more sites may be appropriately linked to give two fragments. The three-dimensional structures of various UDG proteins are known, and those of ordinary skill in the art can appropriately select the split sites based on such information. For example, yeast UNG1 can be split between the 258th amino acid and 259th amino acid from the N-terminal to give N-terminal side fragment (1-258) and C-terminal side fragment (259-359).

As mentioned above, in the base excision repair (BER) mechanism, when a base is excised by DNA glycosylase, AP endonuclease puts a nick in the abasic site (AP site), and exonuclease completely excises the AP site. When the AP site is excised, DNA polymerase produces a new base by using the base of the opposing strand as a template, and DNA ligase finally seals the nick to complete the repair. Mutant AP endonuclease that has lost the enzyme activity but maintains the binding capacity to the AP site is known to competitively inhibit BER. Therefore, when the mutant AP endonuclease is contacted with double-stranded DNA together with DNA glycosylase, the repair of the AP site by endogenous BER mechanism in the host cell is inhibited, and the frequency of repair errors, namely, efficiency of mutation induction, is improved. For example, the efficiency of mutation induction into thymine in mutant yeast UNG1 having lower TDG activity (Y164G) as compared to CDG activity (N222D) can be improved by using mutant AP endonuclease in combination. While the derivation of AP endonuclease is not particularly limited, for example, AP endonuclease derived from Escherichia coli, yeast, mammal (e.g., human, mouse, swine, bovine, horse, monkey etc.) and the like can be used. For example, UniprotKB No. P27695 can be referred to for the amino acid sequence of human Ape1. Examples of the mutant AP endonuclease that has lost the enzyme activity but maintains the binding capacity to the AP site include proteins having mutated activity site or mutated Mg (cofactor)-binding site. For example, E96Q, Y171A, Y171F, Y171H, D210N, D210A, N212A and the like can be mentioned for human Ape1.

In one embodiment of the present disclosure, the complex of the present disclosure in which a nucleic acid sequence-recognizing module and a DNA glycosylase are bound may consist of mutants of Cas9 with inactivated nuclease activity and abasic enzyme UNG. In plants, the combination of nCas9 and UNG can induce various mutations centered on large defects around the target. In combination with dCas9, it is possible to induce a wide variety of mutations, including short deletions as well as point mutations around the target, and also improve the viability of transformants. By selectively using these, it can be used as a semi-rational evolutionary method that induces various mutations in specific regions of the plant genome.

In one embodiment of the present disclosure, a target nucleotide sequence in a double-stranded DNA to be recognized by the nucleic acid sequence-recognizing module in the complex of the present disclosure is not particularly limited as long as the module specifically binds to, and may be any sequence in the double-stranded DNA. The length of the target nucleotide sequence only needs to be sufficient for specific binding of the nucleic acid sequence-recognizing module. For example, when mutation is introduced into a particular site in the genomic DNA of a mammal, it is not less than 12 nucleotides, preferably not less than 15 nucleotides, more preferably not less than 17 nucleotides, according to the genome size thereof. While the upper limit of the length is not particularly limited, it is preferably not more than 25 nucleotides, more preferably not more than 22 nucleotides.

In one embodiment of the present disclosure, as the nucleic acid sequence-recognizing module in the complex of the present invention, CRISPR-Cas system in which Cas does not have at least one DNA cleavage ability (CRISPR-mutant Cas), CRISPR-Cas system in which Cas does not have both of DNA cleavage ability (CRISPR-mutant Cas), zinc finger motif, TAL effector and PPR motif and the like, as well as a fragment containing a DNA binding domain of a protein that specifically binds to DNA, such as restriction enzyme, transcription factor, RNA polymerase and the like, and free of a DNA double strand cleavage ability and the like can be used, but the module is not limited thereto. Preferably, CRISPR-mutant Cas, zinc finger motif, TAL effector, PPR motif and the like can be mentioned.

A zinc finger motif is constituted by linkage of 3 - 6 different Cys2His2 type zinc finger units (1 finger recognizes about 3 bases), and can recognize a target nucleotide sequence of 9 - 18 bases. A zinc finger motif can be produced by a known method such as Modular assembly method (Nat Biotechnol

(2002) 20: 135-141), OPEN method (Mol Cell (2008) 31: 294-301), CoDA method (Nat Methods (2011) 8: 67-69), Escherichia coli one-hybrid method (Nat Biotechnol (2008) 26:695-701) and the like. Various documents can be referred to as for the detail of the zinc finger motif production.

A TAL effector has a module repeat structure with about 34 amino acids as a unit, and the 12th and 13th amino acid residues (called RVD) of one module determine the binding stability and base specificity. Since each module is highly independent, TAL effector specific to a target nucleotide sequence can be produced by simply connecting the module. For TAL effector, a production method utilizing an open resource (REAL method (Curr Protoc Mol Biol (2012) Chapter 12: Unit 12.15), FLASH method (Nat Biotechnol (2012) 30: 460-465), and Golden Gate method (Nucleic Acids Res (2011) 39: e82) etc.) have been established, and a TAL effector for a target nucleotide sequence can be designed comparatively conveniently. Various documents can be referred to as for the detail of the production of TAL effector.

PPR motif is constituted such that a particular nucleotide sequence is recognized by a continuation of PPR motifs each consisting of 35 amino acids and recognizing one nucleic acid base, and recognizes a target base only by 1, 4 and ii(-2) amino acids of each motif. Motif constitution has no dependency, and is free of interference of motifs on both sides. Therefore, like TAL effector, a PPR protein specific to the target nucleotide sequence can be produced by simply connecting PPR motifs. Various documents can be referred to as for the detail of the production of PPR motif.

When a fragment of restriction enzyme, transcription factor, RNA polymerase and the like is used, since the DNA binding domains of these proteins are well known, a fragment containing the domain and free of a DNA double strand cleavage ability can be easily designed and constructed.

As mentioned above, DNA glycosylase used for the complex of the present invention is preferably mutant UDG conferred with CDG activity or TDG activity, more preferably UNG, and it needs to be sufficiently low in the reactivity with a double-stranded DNA so that CDG activity or TDG activity will not act on anywhere in the targeted site. Therefore, the targeted site is desirably in the state of single stranded DNA, or relaxed DNA structure resulting from unwinding of at least firm double helix structure, which enables the DNA glycosylase to act efficiently when brought into contact with DNA glycosylase. When CRISPR-Cas system is used as the nucleic acid sequence-recognizing module, guide RNA complementary to the target nucleotide sequence recognizes the sequence of the object double-stranded DNA and specifically forms a hybrid with the target nucleotide sequence, whereby the targeted site becomes the state of single strand or unwound double helix structure (relaxed double-stranded) state. Therefore, DNA glycosylase with sufficiently low reactivity with a double-stranded DNA selectively acts on cytosine or thymine in the targeted site and can excise the base. On the other hand, when zinc finger motif, TAL effector, PPR motif and the like are used as the nucleic acid sequence-recognizing module, since the module itself does not have a function to change the structure of double-stranded DNA (cause distortion of double helix structure), it is desirable to contact the complex of the present disclosure in combination with a factor (e.g., gyrase, topoisomerase, helicase etc.), which changes the structure of the double-stranded DNA, with the double-stranded DNA of interest.

Any of the above-mentioned nucleic acid sequence-recognizing module can be provided as a fusion protein with the above-mentioned DNA glycosylase, or a protein binding domain such as SH3 domain, PDZ domain, GK domain, GB domain and the like and a binding partner thereof may be fused with a nucleic acid sequence-recognizing module and a DNA glycosylase, respectively, and provided as a protein complex via an interaction of the domain and a binding partner thereof. Alternatively, a nucleic acid sequence-recognizing module and a DNA glycosylase may be each fused with intein, and they can be linked by ligation after protein synthesis.

The complex of the present disclosure (including fusion protein) wherein a nucleic acid sequence-recognizing module and DNA glycosylase are bonded is contacted with a double-stranded DNA (e.g., genomic DNA) by introducing the complex or a nucleic acid encoding the complex into a cell having the double-stranded DNA of interest. In consideration of the introduction and expression efficiency, it is desirable to introduce the complex into the cell in the form of a nucleic acid encoding the complex, rather than the complex itself, and allow for expression of the complex in the cell. Also when mutant AP endonuclease, A20 protein and the like are used in combination, it is desirable to introduce them into the cell in the form of a nucleic acid encoding them, and allow for expression of the complex in the cell.

Therefore, the nucleic acid sequence-recognizing module and the DNA glycosylase are preferably prepared as a nucleic acid encoding a fusion protein thereof, or in a form capable of forming a complex in a host cell after translation into a protein by utilizing a binding domain, intein and the like, or as a nucleic acid encoding each of them. The nucleic acid here may be a DNA or an RNA. When it is a DNA, it is preferably a double-stranded DNA, and provided in the form of an expression vector disposed under regulation of a functional promoter in a host cell. When it is an RNA, it is preferably a single stranded RNA.

When nucleic acid sequence-recognizing module and DNA glycosylase are each split into two fragments, the fragments of either of them are respectively linked to the fragments of the other to provide two partial complexes, for example, a DNA encoding the N-terminal side fragment and a DNA encoding the C-terminal side fragment of the nucleic acid sequence-recognizing module are respectively prepared by the PCR method using suitable primers; and a DNA encoding the N-terminal side fragment and a DNA encoding the C-terminal side fragment of DNA glycosylase are prepared in the same manner and, for example, the DNAs encoding the N-terminal side fragments, and the DNAs encoding the C-terminal side fragments are linked to each other by a conventional method, whereby a DNA encoding the two partial complexes can be produced. Alternatively, a DNA encoding the N-terminal side fragment of the nucleic acid sequence-recognizing module and a DNA encoding the C-terminal side fragment of the DNA glycosylase are linked; and a DNA encoding the N-terminal side fragment of the DNA glycosylase and a DNA encoding the C-terminal side fragment of the nucleic acid sequence-recognizing module are linked, whereby a DNA encoding the two partial complexes can also be produced. The combination of the fragments to be linked is not particularly limited as long as a complex of the functional nucleic acid sequence-recognizing module and the functional DNA glycosylase is reconstituted when the two partial complexes are associated. The two partial complexes are not only expressed as separate molecules, but may also be expressed as a single fusion protein by linking nucleic acids encoding them directly or via a suitable linker, which protein forms a complex of the functional nucleic acid sequence-recognizing module and the functional DNA glycosylase by intramolecular association.

Since the complex of the present invention wherein a nucleic acid sequence-recognizing module and a DNA glycosylase are bonded does not accompany double-stranded DNA breaks (DSB), genome editing with low toxicity is possible, and the genetic modification method of the present disclosure can be applied to a wide range of biological materials. Therefore, the cells to be introduced with nucleic acid encoding nucleic acid sequence-recognizing module and/or DNA glycosylase can encompass cells of any species, from bacterium of Escherichia coli and the like which are prokaryotes, cells of microorganism such as yeast and the like which are lower eucaryotes, to cells of vertebrata including mammals such as human and the like, and cells of higher eukaryote such as insect, plant and the like.

A DNA encoding a nucleic acid sequence-recognizing module such as zinc finger motif, TAL effector, PPR motif and the like can be obtained by any method mentioned above for each module. A DNA encoding a sequence-recognizing module of restriction enzyme, transcription factor, RNA polymerase and the like can be cloned by, for example, synthesizing an oligoDNA primer covering a region encoding a desired part of the protein (part containing DNA binding domain) based on the cDNA sequence information thereof, and amplifying by the RT-PCR method using, as a template, the total RNA or mRNA fraction prepared from the protein-producing cells.

A DNA encoding DNA glycosylase can also be cloned similarly by synthesizing an oligoDNA primer based on the cDNA sequence information thereof, and amplifying by the RT-PCR method using, as a template, the total RNA or mRNA fraction prepared from the enzyme-producing cells. For example, a DNA encoding UNG1 of yeast can be cloned by designing suitable primers for the upstream and downstream of CDS based on the cDNA sequence (accession No. NM_001182379) registered in the NCBI database, and cloning from yeast-derived mRNA by the RT-PCR method.

A nucleic acid encoding DNA glycosylase with sufficiently low reactivity with a double-stranded DNA can be obtained by a known site specific mutagenesis method by using the obtained cDNA as a template, and introducing a mutation imparting CDG activity, TDG activity or 5-mCDG activity, and a mutation that decreases reactivity with a double-stranded DNA. When DNA glycosylase with natively sufficiently low reactivity with a double-stranded DNA, such as vvUDG and the like, only a mutation imparting CDG activity, TDG activity or 5-mCDG activity can be introduced.

The cloned DNA may be directly, or after digestion with a restriction enzyme when desired, or after addition of a suitable linker (e.g., GS linker, GGGAR linker etc.), spacer (e.g., FLAG sequence etc.) and/or a nuclear localization signal (NLS) (each organelle localization signal when the object double-stranded DNA is mitochondria or chloroplast DNA), ligated with a DNA encoding a nucleic acid sequence-recognizing module to prepare a DNA encoding a fusion protein. Since UNG1 and UNG2 each have a mitochondria localization signal and a nuclear localization signal on the N-terminal, they may also be utilized as they are. Alternatively, for example, when UNG1 is used for nucleotide modification targeting nuclear genomic DNA, it is possible to remove the mitochondria localization signal and separately link a nuclear localization signal.

Alternatively, a DNA encoding a nucleic acid sequence-recognizing module, and a DNA encoding a DNA glycosylase may be each fused with a DNA encoding a binding domain or a binding partner thereof, or both DNAs may be fused with a DNA encoding a separation intein, whereby the nucleic acid sequence-recognizing conversion module and the DNA glycosylase are translated in a host cell to form a complex. In these cases, a linker and/or a nuclear localization signal can be linked to a suitable position of one of or both DNAs when desired.

A DNA encoding a nucleic acid sequence-recognizing module and a DNA encoding a DNA glycosylase can be obtained by chemically synthesizing the DNA strand, or by connecting synthesized partly overlapping oligoDNA short strands by utilizing the PCR method and the Gibson Assembly method to construct a DNA encoding the full length thereof. The advantage of constructing a full-length DNA by chemical synthesis or a combination of PCR method or Gibson Assembly method is that the codons to be used can be designed in full-length CDS according to the host into which the DNA is introduced. In the expression of a heterologous DNA, the protein expression level is expected to increase by converting the DNA sequence thereof to codons highly frequently used in the host organism. As the data of codon use frequency in host to be used, for example, the genetic code use frequency database (http://www.kazusa.or.jp/codon/index.html) disclosed in the home page of Kazusa DNA Research Institute can be used, or documents showing the codon use frequency in each host may be referred to. By reference to the obtained data and the DNA sequence to be introduced, codons showing low use frequency in the host from among those used for the DNA sequence may be converted to codons coding the same amino acid and showing high use frequency.

An expression vector containing a DNA encoding a nucleic acid sequence-recognizing module and/or a DNA glycosylase can be produced, for example, by linking the DNA to the downstream of a promoter in a suitable expression vector.

As the expression vector, Escherichia coli-derived plasmids (e.g., pBR322, pBR325, pUC12, pUC13); Bacillus subtilis-derived plasmids (e.g., pUB110, pTP5, pC194); yeast-derived plasmids (e.g., pSH19, pSH15); insect cell expression plasmids (e.g., pFast-Bac); animal cell expression plasmids (e.g., pA1-11, pXT1, pRc/CMV, pRc/RSV, pcDNAI/Neo); bacteriophages such as λphage and the like; insect virus vectors such as baculovirus and the like (e.g., BmNPV, AcNPV); animal virus vectors such as retrovirus, vaccinia virus, adenovirus and the like, and the like are used.

As the promoter, any promoter appropriate for a host to be used for gene expression can be used. In a conventional method using DSB, since the survival rate of the host cell sometimes decreases markedly due to the toxicity, it is desirable to increase the number of cells by the start of the induction by using an inductive promoter. However, since sufficient cell proliferation can also be afforded by expressing the complex of the present disclosure, a constitution promoter can also be used without limitation.

In one embodiment of the present disclosure, since the method of the present disclosure aims to produce a plant cell with modified targeted sites of double-stranded DNA, a plant cell can be preferably used as a host thereof. When the host is a plant cell, CaMV35S promoter, CaMV19S promoter, NOS promoter, and the like are preferred.

In other aspect of the present disclosure, the method of the present disclosure can produce cells in which the targeted site of the double-stranded DNA is modified. In this case, animal cells and the like can also be used as the hosts thereof. For example, when the host is an animal cell, SRα promoter, SV40 promoter, LTR promoter, CMV (cytomegalovirus) promoter, RSV (Rous sarcoma virus) promoter, MoMuLV (Moloney mouse leukemia virus) LTR, HSV-TK (simple herpes virus thymidine kinase) promoter and the like are used. When the host is Escherichia coli, trp promoter, lac promoter, recA promoter, λPL promoter, lpp promoter, T7 promoter and the like are preferred. When the host is genus Bacillus, SPO1 promoter, SPO2 promoter, penP promoter and the like are preferred. When the host is a yeast, Gal1/10 promoter, PHOS promoter, PGK promoter, GAP promoter, ADH promoter and the like are preferred. When the host is an insect cell, polyhedrin promoter, P10 promoter and the like are preferred.

As the expression vector, besides those mentioned above, one containing enhancer, splicing signal, terminator, polyA addition signal, a selection marker such as drug resistance gene, auxotrophic complementary gene and the like, replication origin and the like on demand can be used.

An RNA encoding a nucleic acid sequence-recognizing module and/or a DNA glycosylase can be prepared by, for example, transcription to mRNA in a known in vitro transcription system by using a vector encoding DNA encoding the above-mentioned nucleic acid sequence-recognizing module and/or a DNA glycosylase as a template.

A complex of a nucleic acid sequence-recognizing module and a DNA glycosylase can be intracellularly expressed by introducing an expression vector containing a DNA encoding a nucleic acid sequence-recognizing module and/or a DNA glycosylase into a host cell, and culturing the host cell.

In one embodiment, as the host cell, plant cell, genus Escherichia, genus Bacillus, yeast, insect cell, insect, animal cell, and the like can be used. As the plant cells, suspension cultured cells prepared from various plants (e.g., grains such as rice, wheat, maize, bean, cotton, barley, hemp, sesame, buckwheat and the like, commercial crops such as tomato, cucumber, egg plant, banana, coffee, lettuce and the like, garden plants such as carnation, Eustoma russellianum, safflower, sunflower, rose, Japanese yew and the like, experiment plants such as tobacco, Arabidopsis thaliana and the like, and the like), callus, protoplast, leaf segment, root segment and the like can be used.

In another embodiment, as the genus Escherichia, Escherichia coli K12·DH1 [Proc. Natl. Acad. Sci. USA, 60, 160 (1968)], Escherichia coli JM103 [Nucleic Acids Research, 9, 309 (1981)], Escherichia coli JA221 [Journal of Molecular Biology, 120, 517 (1978)], Escherichia coli HB101 [Journal of Molecular Biology, 41, 459 (1969)], Escherichia coli C600 [Genetics, 39, 440 (1954)] and the like are used. As the genus Bacillus, Bacillus subtilis MI114 [Gene, 24, 255 (1983)], Bacillus subtilis 207-21 [Journal of Biochemistry, 95, 87 (1984)] and the like are used. As the yeast, Saccharomyces cerevisiae AH22, AH22R⁻, NA87-11A, DKD-5D, 20B-12, Schizosaccharomyces pombe NCYC1913, NCYC2036, Pichia pastoris KM71 and the like are used. As the insect cell when the virus is AcNPV, cells of cabbage armyworm larva-derived established line (Spodoptera frugiperda cell; Sf cell), MG1 cells derived from the mid-intestine of Trichoplusia ni, High Five^{™} cells derived from an egg of Trichoplusia ni, Mamestra brassicae-derived cells, Estigmena acrea-derived cells and the like are used. When the virus is BmNPV, cells of Bombyx mori-derived established line (Bombyx mori N cell; BmN cell) and the like are used as insect cells. As the Sf cell, for example, Sf9 cell (ATCC CRL1711), Sf21 cell [all above, In Vivo, 13, 213-217 (1977)] and the like are used. As the insect, for example, larva of Bombyx mori, Drosophila, cricket and the like are used [Nature, 315, 592 (1985)]. As the animal cell, cell lines such as monkey COS-7 cell, monkey Vero cell, Chinese hamster ovary (CHO) cell, dhfr gene-deficient CHO cell, mouse L cell, mouse AtT-20 cell, mouse myeloma cell, rat GH3 cell, human FL cell and the like, pluripotent stem cells such as iPS cell, ES cell and the like of human and other mammals, and primary cultured cells prepared from various tissues are used. Furthermore, zebrafish embryo, Xenopus oocyte, and the like can also be used.

All the above-mentioned host cells may be haploid (monoploid), or polyploid (e.g., diploid, triploid, tetraploid and the like). In the conventional mutation introduction methods, mutation is, in principle, introduced into only one homologous chromosome to produce a hetero gene type. Therefore, desired phenotype is not expressed unless dominant mutation occurs, and homozygousness inconveniently requires labor and time. In contrast, according to the present disclosure, since mutation can be introduced into any allele on the homologous chromosome in the genome, desired phenotype can be expressed in a single generation even in the case of recessive mutation, which is extremely useful since the problem of the conventional method can be solved.

An expression vector can be introduced by a known method (e.g., lysozyme method, competent method, PEG method, CaCl₂ coprecipitation method, electroporation method, the microinjection method, the particle gun method, lipofection method, Agrobacterium method, Floral dip method, and the like) according to the kind of the host.

For example, when a plant cell is used as a host, an Agrobacterium-mediated method, a method of directly introducing into cells, and the like can be used. As an Agrobacterium-mediated method, the method of Nagel et al. (Nagel et al. (1990), Microbiol. Lett., 67, 325) can be used. This method involves first transforming Agrobacterium by electroporation with, for example, an expression vector appropriate for the plant, and then introducing the transformed Agrobacterium into plant cells by the method described in Gelvin et al. (edited by Gelvin et al.(1994), Plant Molecular Biology Manual (Kluwer Academic Press Publishers)).

In another embodiment, when Escherichia coli is used, for example, it can be transformed according to the methods described in, for example, Proc. Natl. Acad. Sci. USA, 69, 2110 (1972), Gene, 17, 107 (1982) and the like. For the genus Bacillus, a vector can be introduced according to the methods described in, for example, Molecular & General Genetics, 168, 111 (1979) and the like. When yeast is used, a vector can be introduced according to the methods described in, for example, Methods in Enzymology, 194, 182-187 (1991), Proc. Natl. Acad. Sci. USA, 75, 1929 (1978) and the like. When insect cell or insect is used, a vector can be introduced according to the methods described in, for example, Bio/Technology, 6, 47-55 (1988) and the like. When animal cell is used, a vector can be introduced according to the methods described in, for example, Cell Engineering additional volume 8, New Cell Engineering Experiment Protocol, 263-267 (1995) (published by Shujunsha), and Virology, 52, 456 (1973).

A cell introduced with a vector can be cultured according to a known method according to the kind of the host.

As a medium for culturing a plant cell, for example, MS medium, LS medium, B5 medium and the like are used. The pH of the medium is preferably about 5 - about 8. The culture is performed at generally about 20°C - about 30°C. Where necessary, aeration and stirring may be performed.

In another embodiment, when Escherichia coli or genus Bacillus is cultured, a liquid medium is preferable as a medium to be used for the culture. The medium preferably contains a carbon source, nitrogen source, inorganic substance and the like necessary for the growth of the transformant. Examples of the carbon source include glucose, dextrin, soluble starch, sucrose and the like; examples of the nitrogen source include inorganic or organic substances such as ammonium salts, nitrate salts, maize steep liquor, peptone, casein, meat extract, soybean cake, potato extract and the like; and examples of the inorganic substance include calcium chloride, sodium dihydrogen phosphate, magnesium chloride and the like. The medium may contain yeast extract, vitamins, growth promoting factor and the like. The pH of the medium is preferably about 5 - about 8.

As a medium for culturing Escherichia coli, for example, M9 medium containing glucose, casamino acid [Journal of Experiments in Molecular Genetics, 431-433, Cold Spring Harbor Laboratory, New York 1972] is preferable. Where necessary, for example, agents such as 3β-indolylacrylic acid may be added to the medium to ensure an efficient function of a promoter. Escherichia coli is cultured at generally about 15 - about 43°C. Where necessary, aeration and stirring may be performed.

The genus Bacillus is cultured at generally about 30 - about 40°C. Where necessary, aeration and stirring may be performed.

Examples of the medium for culturing yeast include Burkholder minimum medium [Proc. Natl. Acad. Sci. USA, 77, 4505 (1980)], SD medium containing 0.5% casamino acid [Proc. Natl. Acad. Sci. USA, 81, 5330 (1984)] and the like. The pH of the medium is preferably about 5 - about 8. The culture is performed at generally about 20°C - about 35°C. Where necessary, aeration and stirring may be performed.

As a medium for culturing an insect cell or insect, for example, Grace's Insect Medium [Nature, 195, 788 (1962)] containing an additive such as inactivated 10% bovine serum and the like as appropriate and the like are used. The pH of the medium is preferably about 6.2 - about 6.4. The culture is performed at generally about 27°C. Where necessary, aeration and stirring may be performed.

As a medium for culturing an animal cell, for example, minimum essential medium (MEM) containing about 5 - about 20% of fetal bovine serum [Science, 122, 501 (1952)], Dulbecco's modified Eagle medium (DMEM) [Virology, 8, 396 (1959)], RPMI 1640 medium [The Journal of the American Medical Association, 199, 519 (1967)], 199 medium [Proceeding of the Society for the Biological Medicine, 73, 1 (1950)] and the like are used. The pH of the medium is preferably about 6 - about 8. The culture is performed at generally about 30°C - about 40°C. Where necessary, aeration and stirring may be performed.

As mentioned above, a complex of a nucleic acid sequence-recognizing module and a DNA glycosylase can be expressed intracellularly.

An RNA encoding a nucleic acid sequence-recognizing module and/or a DNA glycosylase can be introduced into a host cell by microinjection method, lipofection method and the like. RNA introduction can be performed once or repeated multiple times (e.g., 2 - 5 times) at suitable intervals.

When a complex of a nucleic acid sequence-recognizing module and a DNA glycosylase is expressed by an expression vector or RNA molecule introduced into the cell, the nucleic acid sequence-recognizing module specifically recognizes and binds to a target nucleotide sequence in the double-stranded DNA (e.g., genomic DNA) of interest and, due to the action of the DNA glycosylase linked to the nucleic acid sequence-recognizing module, base excision reaction occurs in the sense strand or antisense strand of the targeted site (whole or partial target nucleotide sequence or appropriately adjusted within several hundred bases including the vicinity thereof) and an abasic site (AP site) is produced in one of the strands of the double-stranded DNA. Then, the base excision repair (BER) system in the cell operates, AP endonuclease first recognizes the AP site and cleaves the phosphoric acid bond in one of DNA strand, and exonuclease removes nucleotide subjected to base excision. Then, DNA polymerase inserts a new nucleotide by using the opposing strand DNA as a template and finally DNA ligase repairs the joint. Various mutations are introduced by a repair miss occurring at any stage of this BER. As mentioned above, the BER mechanism in the cell is inhibited, and the frequency of repair miss, and thus, efficiency of mutation induction can be improved by using a mutant AP endonuclease which lost enzyme activity but retains binding capacity to AP site in combination.

Since the CRISPR-Cas system recognizes the object double-stranded DNA sequence by a guide RNA complementary to the target nucleotide sequence, any sequence can be targeted by simply synthesizing an oligoDNA capable of specifically forming a hybrid with the target nucleotide sequence. Moreover, at the targeted site, since the double-stranded DNA is unwound to generate a region having a single stranded structure, and a region adjacent thereto which has a structure of relaxed double-stranded DNA, DNA glycosylase can be made to act efficiently in a targeted site-specific manner, without combining factors that change the structure of double-stranded DNA. Therefore, in a more preferable embodiment of the present disclosure, a CRISPR-Cas system in which Cas does not have at least one DNA cleavage ability (CRISPR-mutant Cas) or a CRISPR-Cas system in which Cas does not have both of DNA cleavage ability (CRISPR-mutant Cas) can be preferably used as a nucleic acid sequence-recognizing module.

The nucleic acid sequence-recognizing module of the present disclosure using CRISPR-mutant Cas is provided as a complex of an RNA molecule consisting of a guide RNA complementary to the target nucleotide sequence and tracrRNA necessary for recruiting mutant Cas protein, and a mutant Cas protein.

The Cas protein to be used in the present disclosure is not particularly limited as long as it belongs to the CRISPR system, and preferred is Cas9. Examples of Cas9 include, but are not limited to, Streptococcus pyogenes-derived Cas9 (SpCas9), Streptococcus thermophilus-derived Cas9 (StCas9) and the like. Preferred is SpCas9. AS a mutant Cas to be used in the present disclosure, any of Cas in which Cas is not capable of cleaving both strands of the double-stranded DNA and Cas having nickase activity in which Cas is not capable of cleaving only one strand can be used. For example, in the case of SpCas9, a D10A mutant in which the 10th Asp residue is converted to an Ala residue and lacking cleavage ability of a strand opposite to the strand forming a complementary strand with a guide RNA, or H840A mutant in which the 840th His residue is converted to an Ala residue and lacking cleavage ability of strand complementary to guide RNA, or a double mutant thereof can be used, and other mutant Cas can be used similarly.

DNA glycosylase is provided as a complex with mutant Cas by a method similar to the coupling scheme with the above-mentioned zinc finger and the like. Alternatively, a DNA glycosylase and mutant Cas can also be bound by utilizing an RNA scaffold of RNA aptamers MS2F6, PP7 and the like, and the binding proteins thereto. Guide RNA forms a complementary strand with the target nucleotide sequence, mutant Cas is recruited by the tracrRNA attached and mutant Cas recognizes DNA cleavage site recognition sequence PAM (protospacer adjacent motif) (when SpCas9 is used, PAM is 3 bases of NGG (N is any base), and, theoretically, any position on the genome can be targeted), but cannot cleave one or both DNAs, and, due to the action of the DNA glycosylase linked to the mutant Cas, base excision occurs in the targeted site (appropriately adjusted within several hundred bases including whole or partial target nucleotide sequence) and an AP site occurs in the double-stranded DNA. Various mutations are introduced due to the errors made by the BER system of the cell which tries to repair it.

Even when CRISPR-mutant Cas is used as a nucleic acid sequence-recognizing module, a nucleic acid sequence-recognizing module and a DNA glycosylase are desirably introduced, in the form of a nucleic acid encoding same, into a cell having a double-stranded DNA of interest, similar to when zinc finger and the like are used as a nucleic acid sequence-recognizing module.

A DNA encoding Cas can be cloned by a method similar to the above-mentioned method for a DNA encoding a DNA glycosylase, from a cell producing the enzyme. A mutant Cas can be obtained by introducing a mutation to convert an amino acid residue of the site important for the DNA cleavage activity (e.g., 10th Asp residue and 840th His residue for Cas9, though not limited thereto) to other amino acid, into a DNA encoding cloned Cas, by a site specific mutagenesis method known per se.

Alternatively, a DNA encoding mutant Cas can also be constructed as a DNA showing codon usage suitable for expression in a host cell to be used, by a method similar to those mentioned above for a DNA encoding a nucleic acid sequence-recognizing module and a DNA encoding a DNA glycosylase, and by a combination of chemical synthesis or PCR method or Gibson Assembly method.

In one embodiment, a DNA encoding a mutant Cas and a DNA encoding a DNA glycosylase may be linked to allow for expression as a fusion protein, or designed to be separately expressed using a binding domain, intein and the like, and form a complex in a host cell via protein-protein interaction or protein ligation. Alternatively, a design may be employed in which a DNA encoding mutant Cas and a DNA encoding DNA glycosylase are each split into two fragments at suitable split site, either fragments are linked to each other directly or via a suitable linker to express a complex as two partial complexes, which are associated and refolded in the cell to reconstitute functional mutant Cas having a particular nucleic acid sequence recognition ability, and a functional DNA glycosylase having a base excision reaction catalyst activity is reconstituted when the mutant Cas is bonded to the target nucleotide sequence. For example, a DNA encoding the N-terminal side fragment and a DNA encoding the C-terminal side fragment of mutant Cas are respectively prepared by the PCR method by using suitable primers; a DNA encoding the N-terminal side fragment and a DNA encoding the C-terminal side fragment of DNA glycosylase are prepared in the same manner; for example, the DNAs encoding the N-terminal side fragments are linked to each other, and the DNAs encoding the C-terminal side fragments are linked to each other by a conventional method, whereby a DNA encoding two partial complexes can be produced. Alternatively, a DNA encoding the N-terminal side fragment of mutant Cas and a DNA encoding the C-terminal side fragment of DNA glycosylase are linked; and a DNA encoding the N-terminal side fragment of DNA glycosylase and a DNA encoding the C-terminal side fragment of mutant Cas are linked, whereby a DNA encoding two partial complexes can also be produced. Respective partial complexes may be linked to allow for expression as a fusion protein, or designed to be separately expressed using a binding domain, intein and the like, and form a complex in a host cell via protein-protein interaction and protein ligation. Two partial complexes may be linked to be expressed as a fusion protein. The split site of the mutant Cas is not particularly limited as long as the two split fragments can be reconstituted such that they recognize and bind to the target nucleotide sequence, and it may be split at one site to provide N-terminal side fragment and C-terminal side fragment, or not less than 3 fragments obtained by splitting at two or more sites may be appropriately linked to give two fragments. The three-dimensional structures of various Cas proteins are known, and those of ordinary skill in the art can appropriately select the split site based on such information. For example, since the region consisting of the 94th to the 718th amino acids from the N terminus of SpCas9 is a domain (REC) involved in the recognition of the target nucleotide sequence and guide RNA, and the region consisting of the 1099th amino acid to the C-terminal amino acid is the domain (PI) involved in the interaction with PAM, the N-terminal side fragment and the C-terminal side fragment can be split at any site in REC domain or PI domain, preferably in a region free of a structure (e.g., between 204th and 205th amino step from the N-terminal (204..205), between 535th and 536th amino acids from the N-terminal (535..536) and the like) (see, for example, Nat Biotechnol. 33(2): 139-142 (2015)).

The obtained DNA encoding a mutant Cas and/or a DNA glycosylase can be inserted into the downstream of a promoter of an expression vector similar to the one mentioned above, according to the host. On the other hand, a DNA encoding guide RNA and tracrRNA can be obtained by designing an oligoDNA sequence linking guide RNA sequence complementary to the target nucleotide sequence and known tracrRNA sequence and chemically synthesizing using a DNA/RNA synthesizer. While a DNA encoding guide RNA and tracrRNA can also be inserted into an expression vector similar to the one mentioned above, according to the host. As the promoter, pol III system promoter (e.g., SNR6, SNR52, SCR1, RPR1, U6, H1 promoter etc.) and terminator (e.g., T6 sequence) are preferably used.

An RNA encoding mutant Cas and/or a DNA glycosylase can be prepared by, for example, transcription to mRNA in a vitro transcription system known per se by using a vector encoding the above-mentioned mutant Cas and/or DNA encoding a DNA glycosylase as a template.

Guide RNA-tracrRNA can be obtained by designing an oligoDNA sequence linking a sequence complementary to the target nucleotide sequence and known tracrRNA sequence and chemically synthesizing using a DNA/RNA synthesizer.

A DNA or RNA encoding mutant Cas and/or a DNA glycosylase, guide RNA-tracrRNA or a DNA encoding same can be introduced into a host cell by a method similar to the above, according to the host.

Since conventional artificial nucleases accompany double-stranded DNA breaks (DSB), inhibition of growth and cell death assumedly caused by disordered cleavage of chromosomes (off-target cleavage) occur by targeting a sequence in the genome. The effect thereof is particularly fatal for many microorganisms and prokaryotes, and prevents applicability. In the present disclosure, mutation is introduced not by DNA cleavage but by a base excision reaction on the DNA base, and therefore, drastic reduction of toxicity can be realized.

The modification of the double-stranded DNA in the present disclosure does not prevent occurrence of cleavage of the double-stranded DNA in a site other than the targeted site (appropriately adjusted within several hundred bases including whole or partial target nucleotide sequence). However, one of the advantages of the present disclosure is avoidance of toxicity by off-target cleavage, which is generally applicable to any species. In preferable one embodiment, therefore, the modification of the double-stranded DNA in the present disclosure does not accompany cleavage of DNA strand not only in a targeted site of a given double-stranded DNA but in a site other than same.

In one embodiment of the present disclosure, when sequence-recognizing modules are produced corresponding to the adjacent multiple target nucleotide sequences, and simultaneously used, the mutation introduction can be efficiency increased than by using a single nucleotide sequence as a target. As the effect thereof, similarly mutation induction is realized even when both target nucleotide sequences partly overlap or when the both are apart by about 600 bp. It can occur both when the target nucleotide sequences are in the same direction (target nucleotide sequences are present on the same strand), and when they are opposed (target nucleotide sequence is present on each strand of double-stranded DNA).

The genome sequence modification method of the present disclosure can introduce mutation into almost all cells in which the complex of the present disclosure has been expressed, by selecting a suitable target nucleotide sequence. Thus, insertion and selection of a selection marker gene, which are essential in the conventional genome editing, are not necessary. This dramatically facilitates and simplifies gene manipulation and enlarges the applicability to crop breeding and the like since a recombinant organism with foreign DNA is not produced.

Since the genome sequence modification method of the present disclosure shows extremely high efficiency of mutation induction, and does not require selection by markers, modification of multiple DNA regions at completely different positions as targets can be performed. Therefore, in one preferable embodiment of the present disclosure, two or more kinds of nucleic acid sequence-recognizing modules that specifically bind to different target nucleotide sequences (which may be present in one object gene, or two or more different object genes, which object genes may be present on the same chromosome or different chromosomes) can be used. In this case, each one of these nucleic acid sequence-recognizing modules and DNA glycosylase form a complex. Here, a common DNA glycosylase can be used. For example, when CRISPR-Cas system is used as a nucleic acid sequence-recognizing module, a common complex of a Cas protein and a DNA glycosylase (including fusion protein) is used, and two or more kinds of chimeric RNAs of tracrRNA and each of two or more guide RNAs that respectively form a complementary strand with a different target nucleotide sequence are produced and used as guide RNA-tracrRNAs. On the other hand, when zinc finger motif, TAL effector and the like are used as nucleic acid sequence-recognizing modules, for example, a DNA glycosylase can be fused with a nucleic acid sequence-recognizing module that specifically binds to a different target nucleotide.

To express the complex of the present disclosure in a host cell, as mentioned above, an expression vector containing a DNA encoding the complex, or an RNA encoding the complex is introduced into a host cell. For efficient introduction of mutation, it is desirable to maintain an expression of complex of a given level or above for not less than a given period. From such aspect, it is ensuring to introduce an expression vector (plasmid etc.) autonomously replicatable in a host cell. However, since the plasmid etc. are foreign DNAs, they are preferably removed rapidly after successful introduction of mutation. When mutant AP endonuclease is used in combination, since the mutant enzyme inhibits the BER mechanism in the host cell, it may induce undesirable spontaneous mutations outside the target region. Thus, it is preferable to also remove a plasmid containing a DNA encoding the mutant enzyme promptly after introduction of the desired mutation. Therefore, though subject to change depending on the kind of host cell and the like, for example, the introduced plasmid is desirably removed from the host cell after a lapse of for 6 hr - 2 days from the introduction of an expression vector by using various plasmid removal methods well known in the art.

Alternatively, as long as expression of a complex, which is sufficient for the introduction of mutation, is obtained, it is preferable to introduce mutation into the object double-stranded DNA by transient expression by using an expression vector without autonomous replicatability in a host cell (e.g., vector etc. lacking replication origin that functions in host cell and/or gene encoding protein necessary for replication) or RNA.

### <Production of modified plant cell>

In one embodiment of the present disclosure, the method of the present disclosure is a method for causing a predetermined modification in a plant cell by transfecting a complex in which the above-mentioned nucleic acid sequence-recognizing module and a DNA glycosylase are bound into the plant cell and expressing the complex. Introduction of the complex is not particularly limited as long as the gene encoding the complex is introduced into the plant. The vectors described elsewhere in the present specification can be used to introduce the gene into the desired plant. Examples of the methods for introducing into the target cell include Agrobacterium method, Floral dip method, particle gun (bombardment) method, RNA virus vector method, plasma treatment method, PEG method, electroporation method, and the like.

The methods for introducing mutations into the target genes by genome editing include, for example, a transformation method using Agrobacterium, a method for directly introducing factors for genome editing into protoplasts, and the like. The transformation method using Agrobacterium is a method for introducing a factor for genome editing into plant cells by infecting the plant cells with Agrobacterium bacteria containing a vector that expresses the factors for genome editing. That is, a plant cell or plant tissue isolated from the plant of interest (e.g., lily family plant), incubated with an Agrobacterium bacterium containing a vector that expresses a factor for genome editing, and the factor for genome editing is introduced into the plant cell. Thereafter, cell culture or tissue culture is performed under appropriate culture conditions to produce a genome-edited callus. Thereafter, the genome-edited callus is induced to differentiate to regenerate the genome-edited plant body, whereby a plant body in which the genome of the whole plant body is edited is obtained. The method of directly introducing a factor for genome editing into protoplasts is a method of directly introducing a factor for genome editing into plant cells without using Agrobacterium or a vector. That is, protoplasts are prepared from plant cells derived from a plant of interest, and a factor for genome editing is introduced directly into the protoplasts by the PEG method, particle gun method, or the like. Thereafter, a genome-edited plant can be obtained by incubating the protoplasts under appropriate culture conditions.

The Agrobacterium method is a method using a soil bacterium Agrobacterium tumefaciens of the genus Rhizobium, Rizobium radiobacter, Agrobacteriumrhizogenes, Rizobiumrhizogenes, or the like. The fact that the T-DNA region of the Ti or Ri plasmid possessed by this fungus is inserted into the plant genome by homologous recombination can be utilized. That is, once the target gene is inserted into the T-DNA region, the target gene can be introduced into the plant genome when the plant is infected with Agrobacterium.

The Floral dip method is a method in which the desired gene is introduced into both a cell to be the stamen and a cell to be the pistil by infecting flower buds with Agrobacterium, followed by pollination to introduce the target gene into the plant genome.

The particle gun method is a method of introducing DNA into plant cells by ejecting DNA-coated metal particles such as gold or tungsten as bullets at high speed. High-pressure gas such as helium is mainly used for injecting metal particles.

In the Agrobacterium method, for example, plant cotyledon or callus is infected with Agrobacterium containing a vector containing a gene encoding the above-mentioned complex, antibiotics for eradicating Agrobacterium and antibiotics for selecting gene transfer cells are added to the medium, and sterilization and selection are repeated, whereby the gene can be introduced into the plant.

The vector in this case is not particularly limited as long as it can express the above-mentioned gene in the plant, and various plasmid vectors can be used. For example, CaMV35S promoter, CaMV19S promoter, NOS promoter, pRI201-AN vector (Takara Bio Inc.), pBI vector (Takara Bio Inc.), pRI vector (Takara Bio Inc.), pFAST vector (Thermo Fisher Scientific), pSuperAgro vector (Inplanta Innovations Inc.), and the like can be used.

As a special example of a vector, when a plant cell is transformed using an Agrobacterium-mediated gene transfer method, it is necessary to use a plasmid called a "binary vector" having a nucleotide sequence corresponding to the origin of replication of both E. coli and Agrobacterium, and the T-DNA-derived border sequences (left border and right border) corresponding to the border regions that can be introduced into plants. Examples thereof include, but are not limited to, pBI101 (commercially available from Clontech), pBIN (Bevan, N., Nucleic Acid Research 12, 8711-8721, 1984), pBINPlus (van Engelen, FA et al., Tranegenic Research 4, 288-290, 1995), pTN or pTH (Fukuoka H et.al., Plant Cell Reports 19, 2000), pPZP (Hajdukiewicz P et al., Plant Molecular Biology 25, 989-994, 1994), and the like. In addition, a tobacco mosaic virus vector is also exemplified as a vector that can be used in plants. However, since this type of vector does not introduce the target gene into the plant chromosome, its use is limited, and it can be used in the present disclosure only when the gene-transfected plant does not need to be proliferated through seeds.

### <Production of transformed plant>

In one embodiment of the present disclosure, according to the method of the present disclosure, transformed plant can be obtained by forming callus from a plant into which a gene encoding the above-mentioned complex has been introduced, and differentiating roots and shoots from the callus. A method for forming callus from a plant into which a gene has been introduced and/or a method for differentiating roots and shoots from the callus can be performed by culturing a plant or callus into which the gene has been introduced (particularly plant or callus infected with Agrobacterium having a vector containing the gene) in a callus induction medium. At this time, the callus induction medium may further contain an antibiotic for sterilization and an antibiotic for selecting transformed cells. Examples of antibacterial antibiotics include cefotaxime, moxalactam, meropenem, and the like, and their actions enable efficient differentiation of callus into roots and shoots.

As the medium, 2N6 medium (mixed salt for N6 medium (Sigma-Aldrich Co. LLC.) 4.0 g/L, Casamino acid 300 mg/L, Myo-inocitol 100 mg/L, Nicotinic acid 0.5 mg/L, Pyridoxine HCl 0.5 mg/L, Thiamine HCl 0.5 mg/L, L-Proline 2878 mg/L, Sucrose30.0 g/L, 2,4-D (2,4-dichlorophenoxyacetic acid) 2 mg/L, Gelrite 4.0 g/L, pH 5.8) can be used. A plant piece infected with Agrobacterium is placed on this medium and co-cultivated in the dark at about 25°C for 3 days, after which Agrobacterium is sterilized from the callus after co-cultivation. Thereafter, the sterilized callus can be cultured for 5 days in 2N6NU medium (N6 medium mixed salt[Sigma manufactured by] 4.0 g/L, Casamino acid 300 mg/L, Myo-inocitol 100 mg/L, Nicotinic acid 0.5 mg/L, Pyridoxine HCl 0.5 mg/L, Thiamine HCl 0.5 mg/L, L-Proline 2878 mg/L, Sucrose30.0 g/L, 2,4-D 2 mg/L, Gelrite 4.0 g/L, Vancomycin 100 mg/L, Meropenem 25 mg/L, pH 5.8). After that, as described later, callus can be selected by culturing the plant in a medium containing a drug corresponding to any drug resistance gene used for transformation.

### <Selection of gene-transfected cell>

As a method for selecting cells into which the complex of the present disclosure has been introduced and/or cells into which modification by the complex has been introduced, a method of introducing a vector containing any drug resistance gene together with a transgene into a plant, and then culturing the plant in a medium containing a drug corresponding to the drug resistance gene can be mentioned. As the drug resistance gene, kanamycin resistance gene, hygromycin resistance gene, Bialaphos resistance gene, and the like can be mentioned. As medicaments corresponding to these resistance genes, kanamycin, hygromycin, and Bialaphos can be mentioned. For example, when the neomycin phosphotransferase gene is inserted into the T-DNA region of the vector, selection is performed in a medium containing the antibiotic kanamycin. When a hygromycin-resistant gene (HPT) or a bialaphos-resistant gene (bar) gene is inserted, hygromycin or bialaphos is added to the medium, respectively, and Agrobacterium-infected plants are cultured in a medium supplemented with drugs corresponding to these drug resistance genes, whereby plant cells into which the genes have been introduced can be selected.

Cells introduced with the plant expression vector are selected for drug resistance such as hygromycin resistance, kanamycin resistance, and the like as described above. They can then be redifferentiated into plant tissues, plant organs and/or plants by methods well known in the art. Furthermore, seeds may be obtained from the plant. Expression of the introduced gene can be detected by Northern blotting or PCR. Where necessary, the expression of the protein of the gene product can be confirmed by, for example, Western blot method.

In one embodiment of the present disclosure, when cells having desired properties are selected from the cells into which the above-mentioned complex has been introduced and/or the cells into which the above-mentioned modification has been introduced, cells having properties useful as plants, such as yield, storability, ease of cultivation, high quality, and the like can be selected from cells into which the complex has been introduced as a result of transfection of the cells as described above, or cells in which substitution, deletion, or insertion of one or more nucleotides has occurred in one or more nucleotides at the targeted site of the double-stranded DNA by the action of the complex.

While the present disclosure has been shown to be particularly useful in plants, it can also be used in other organisms. The molecular biology techniques used in the present disclosure are well known and conventionally used in the pertinent field. For example, they are described in Ausubel F.A. et al. ed. (1988), Current Protocols in Molecular Biology, Wiley, New York, NY; Sambrook J et al. (1987) Molecular Cloning: A Laboratory Manual, 2nd Ed. and 3rd edition thereof, Cold Spring Harbor Laboratory Press, Cold Spring Harbor, NY, Jikken Igaku, Extra, "Idenshi donyu & Hatsugen kaiseki jikken hou" YODOSHA CO., LTD., 1997 and the like.

In the method of performing transformation, physical methods include polyethylene glycol method (PEG method), electroporation method, microinjection method, and particle gun method. These methods are highly useful in that they can be applied to both monocotyledonous and dicotyledonous plants. However, the polyethylene glycol method and the electroporation method have problems that protoplasts must be used because the cell wall is an obstacle, and the frequency of integration of the introduced gene into the chromosomal DNA of plant cells is low. The microinjection method using callus or tissue without using protoplasts have many difficulties regarding needle thickness, tissue fixation, and the like. Even the particle gun method using tissues has problems such as mutation appearing in the form of chimera and the like. In addition, in these physical methods, the introduced foreign gene generally tends to be incompletely integrated into the nuclear genome as a multicopy gene. It is known that introduction of multiple copies of a foreign gene tends to cause inactivation of the gene.

On the other hand, methods for introducing an isolated gene using organisms include the Agrobacterium method, the viral vector method, and a method using pollen as a vector. Since these methods use plant callus, tissue or plant bodies for gene transfer without using protoplasts, they have the advantages of not being cultured for a long period of time and being less susceptible to disorders such as somaclonal mutations. Of these methods, the method using pollen as a vector has still few experimental examples, and includes many unknown parts as a plant transformation method. Although the virus vector method has the advantage that the gene to be introduced spreads throughout the virus-infected plant body, it has problems in that the gene is only amplified and expressed in each cell, and there is no guarantee that it will be transmitted to the next generation, and that long DNA fragments cannot be introduced. The Agrobacterium method has many advantages, such as the ability to introduce DNA of about 20 kbp or more into the chromosome without major rearrangement, the low number of copies of the gene to be introduced, and high reproducibility. Since Agrobacterium is outside the host range of monocotyledonous plants such as gramineous plants, introduction of exogenous genes into gramineous plants has heretofore been carried out by physical methods as described above. However, in recent years, the Agrobacterium method has come to be applied to monocotyledonous plants such as rice and the like, for which a culture system has been established, and the Agrobacterium method is currently preferred.

In the introduction of a foreign gene by the Agrobacterium method, when a low-molecular-weight phenolic compound such as acetosyringone synthesized by a plant acts on the Ti plasmid Vir region, the T-DNA region is excised from the Ti plasmid, and integrated into nuclear chromosomal DNA of plant cells through several processes. In dicotyledonous plants, since the plants themselves have a mechanism for synthesizing such phenolic compounds, exogenous genes can be easily introduced by the leaf disk method or the like, and the reproducibility is also high. On the other hand, monocotyledonous plants do not synthesize such phenolic compounds themselves, and production of transformed plants by Agrobacterium has been difficult. However, it is now possible to introduce foreign genes into monocotyledonous plants by adding acetosyringone at the time of Agrobacterium infection.

In the present disclosure, in the transformant, the desired nucleic acid molecule (transgene) may or may not have been introduced into the chromosome. Preferably, the nucleic acid molecule of interest (transgene) is introduced into a chromosome, more preferably into both of the two chromosomes.

Techniques and media known in the art are used for culture, dedifferentiation, differentiation, and regeneration of plant cells, plant tissues, and plants. Examples of such medium include, but are not limited to, Murashige-Skoog (MS) medium, GaMborg B5 (B) medium, White medium, Nitsch&Nitsch (Nitsch) medium, and the like. These media are generally used by adding an appropriate amount of a plant growth regulator (plant hormone) and the like.

In the present specification, in the case of a plant, "redifferentiation" means a phenomenon in which cells in an undifferentiated state differentiate into more differentiated entities such as cells, tissues, or whole organisms having functions. For example, tissue fragments such as cells (leaves, roots, etc.) can be formed by redifferentiation of callus, and organs or plants can be formed by growing these tissue fragments.

Methods for redifferentiating transformants into plants are well known in the art. Examples of such method include, but are not limited to, those described in Rogers et al., Methods in Enzymology 118:627-640 (1986); Tabata et al., Plant Cell Physiol., 28:73-82 (1987); Shaw, Plant Molecular Biology: A practical approach. IRL press (1988); Shimamoto et al., Nature 338:274 (1989); Maliga et al., Methods in Plant Molecular Biology: A laboratory course. Cold Spring Harbor Laboratory Press (1995), and the like. Therefore, those skilled in the art can appropriately use the above-mentioned well-known method according to the target transgenic plant to achieve regeneration. The transgenic plant thus obtained has the target gene introduced therein, and introduction of such genes can be confirmed using the methods described in the present specification, such as Northern blot, Western blot analysis, or other well-known conventional techniques.

### (General technique)

Molecular biological techniques, biochemical techniques, and microbiological techniques used in the present specification are well known and conventionally used in the art and described in, for example, Sambrook J. et al. (1989). Molecular Cloning: A Laboratory Manual, Cold Spring Harbor and 3rd Ed. thereof (2001); Ausubel, F.M. (1987). Current Protocols in Molecular Biology, Greene Pub. Associates and Wiley-Interscience; Ausubel, F.M. (1989). Short Protocols in Molecular Biology: A Compendium of Methods from Current Protocols in Molecular Biology, Greene Pub. Associates and Wiley-Interscience; Innis, M.A. (1990). PCR Protocols: A Guide to Methods and Applications, Academic Press; Ausubel, F.M.

(1992). Short Protocols in Molecular Biology: A Compendium of Methods from Current Protocols in Molecular Biology, Greene Pub. Associates; Ausubel, F.M. (1995). Short Protocols in Molecular Biology: A Compendium of Methods from Current Protocols in Molecular Biology, Greene Pub. Associates; plant regulator Innis, M.A. et al. (1995). PCR Strategies, Academic Press; Ausubel, F.M. (1999). Short Protocols in Molecular Biology: A Compendium of Methods from Current Protocols in Molecular Biology, Wiley, and annual updates; Sninsky, J.J. et al. (1999). PCR Applications: Protocols for Functional Genomics, Academic Press, Jikken Igaku, Extra" Idenshi Donyu & Hatsugen Kaiseki jikken Hou" YODOSHA CO., LTD., 1997 and the like. The relevant portions thereof (which may be all of them) are incorporated herein by reference.

For DNA synthesis technique and nucleic acid chemistry for producing artificially synthesized genes, gene synthesis and fragment synthesis services such as GeneArt, GenScript, Integrated DNA Technologies (IDT), and the like can also be used. In addition, they are described in, for example, Walking, M.J.(1985). Oligonucleotide Synthesis: A Practical Approach, IRL Press; Walking, M.J.(1990). Oligonucleotide Synthesis: A Practical Approach, IRL Press; Eckstein, F.(1991). Oligonucleotides and Analogues: A Practical Approach, IRL Press; Adams, R.L. et al. (1992). The Biochemistry of the Nucleic Acids, Chapman & Hall; Shabarova, Z. et al. (1994). Advanced Organic Chemistry of Nucleic Acids, Weinheim; Blackburn, G.M. et al.(1996). Nucleic Acids in Chemistry and Biology, Oxford University Press; Hermanson, G.T.(I996). Bioconjugate Techniques, Academic Press, and the like. The relevant portions thereof are incorporated herein by reference.

As used herein, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure described hereinafter is based on the Examples. The above descriptions and the following Examples are not provided to limit the present disclosure, but for the sole purpose of exemplification. Thus, the scope of the present disclosure is not limited to the embodiments and Examples specifically described herein and is limited only by the scope of claims.

### [Example]

### (Example 1 : Introduction of mutation by Target-G)

### 1-1. Construction of Target-G expression unit

A maize ubiquitin gene promoter (SEQ ID NO: 1) was used as a constitutive promoter in monocotyledons. The artificial gene fusing the modified Cas9 gene and the DNA uracil glycosylase gene was linked at the downstream of the promoter. The main constituent elements are described below. Cas9 derived from Streptococcus pyogenes was modified into a nuclease completely inactivated form (dCas9) (SEQ ID NO: 2), and nuclear localization signals (ATG GCT CCT AAG AAG AAG CGG AAG GTT GGT ATT CACGGG GTG CCT GCG GCT; SEQ ID NO: 3 (encoding MAPKKKRKVGIHGVPAA), CCCAAG AAG AAG AGG AAG GTG; SEQ ID NO: 4 (encoding PKKKRV)) were added to the both ends. In addition, as a modified Cas9 gene, a partially inactivated nuclease (nCas9) (SEQ ID NO: 5) was also constructed, and a nuclear localization signal (SEQ ID NO: 4) was added to the C-terminal side. Furthermore, 1 copy or 2 copies of the modified UNG1 gene derived from yeast mitochondria is/are linked to the C-terminal side of dCas9 or nCas9 via a linker sequence. In UNG1, the region encoding the mitochondria localization signal (base numbers 1 to 60) is excluded from the wild-type gene, and the N222D mutation (base number 664 a is replaced with g (a664g), the N222D mutation (a replaced by g in base 664 (a664g)) and the R308C mutation (aga replaced by tgt in bases 922-924 (aga922tgt)) were introduced, and the modified form was used (SEQ ID NO: 6). The above-mentioned constituent elements were combined to construct 4 kinds of Target-G unit. Specific structures of respective units were dCas9 linked with one copy of UNG1, "d1" (SEQ ID NO: 7), dCas9 linked with two copies of UNG1, "d2" (SEQ ID NO: 8), nCas9 linked with one copy of UNG1, "n1" (SEQ ID NO: 9), and nCas9 linked with two copies of UNG1, "n2" (SEQ ID NO: 10).

### 1-2. Construction of guide RNA expression unit

A rice RNA polymerase III-dependent U6 promoter (hereinafter OsU6) (SEQ ID NO: 11) was used as a promoter for expressing the guide RNA in monocotyledonous plants. In this Experiment, six target sequences in the rice genome were planed to be modified, and guide RNAs corresponding to each target were designed. The sequences are shown below.
1. ALS A96:CATGGACGCGCCGCCCGGGT (SEQ ID NO: 12)
2. OsFTIP1eQ590:CAGCTGTGGAAACCACCAAT (SEQ ID NO: 13)
3. DL-3:GACCTTGCACTGACTGCAGG (SEQ ID NO: 14)
4. OsClpP5:GCCATGGCCGCGGCTCGTGG (SEQ ID NO: 15)
5. EGFP Mut1:GAGCTGGACGGCGACGTAAA (SEQ ID NO: 16)
6. EGFP Mut2:CATGTGATCGCGCTTCTCGT (SEQ ID NO: 17)
7. mGFP switch:TCTAGAGGATCCGGCCCAGT (SEQ ID NO: 18)
8. ALSP171-R173:CAGGTCCCCCGCCGCATGAT (SEQ ID NO: 19)

In this Experiment, 4 types of units that combined multiple guide RNAs were used. The first unit is "x4" (SEQ ID NO: 20), which is a combination of the above-mentioned guide RNAs 1 to 4, the second is "x6" (SEQ ID NO: 21), which is a combination of 1 to 6, the third is "x5" (SEQ ID NO: 22), which is a combination of 1 to 4 and 7, and the fourth is "x2" (SEQ ID NO: 23), which is a combination of the above-mentioned guide RNAs 1 and 8.

### 1-3. Construction of constitutive expression type Target-G vector

In order to introduce the Target-G system into plant cells, the Target-G expression unit combined with the maize ubiquitin gene promoter and the gRNA expression unit were incorporated into a general binary vector for the Agrobacterium method (pVS1 system). As a positive marker for selecting plant cells in which the Target-G system is integrated into the genome, a hygromycin phosphotransferase (hpt) expression unit linked to the cauliflower mosaic virus 35S promoter (SEQ ID NO: 24) was used. In this Experiment, the following four types of constant expression Target-G vectors were constructed according to the type of mutant Cas9 and the copy number of UNG1 (Fig. 1).
·pOSGd11 (1 copy of UNG1 was linked to dCas9)
·pOSGd21 (2 copies of UNG1 were linked to dCas9)
·pOSGn11 (1 copy of UNG1 was linked to nCas9)
·pOSGn21 (2 copies of UNG1 were linked to nCas9)

A gRNA expression unit (SEQ ID NO: 20) targeting 4 genes on the rice genome was incorporated into pOSGd11 and pOSGd21 to produce "pOSGd11x4" and "pOSGd21x4". In addition, a gRNA expression unit "x2" (SEQ ID NO: 23) targeting two sites in the rice ALS gene region was incorporated into pOSGn21 to produce "pOSGn21x2".

### 1-4. Construction of heat shock inducible type Target-G vector

In order to construct a heat shock inducible type Target-G system, the maize ubiquitin gene promoter of the constitutively expressing Target-G vectors "pOSGd11" and "pOSGd21" was replaced with the rice heat shock protein 17.9C gene promoter (SEQ ID NO: 25) to construct "pOSGd13" and "pOSGd23". Thereafter, gRNA expression unit (SEQ ID NO: 20) targeting 4 genes on the rice genome was incorporated to produce "pOSGd13x4" and "pOSGd23x4". Furthermore, the positive markers for plant cell selection of "pOSGd13" and "pOSGd23" were replaced with the neomycin phosphotransferase II (nptII) expression unit (SEQ ID NO: 26) linked to the rice actin gene promoter to construct "pOSGd13-nptII" and "pOSGd23-nptII". A guide RNA expression unit (SEQ ID NO: 21) targeting 6 locations on the rice genome was incorporated into these to obtain "pOSGd13-nptIIx6" and "pOSGd23-nptIIx6".

### 1-5. Construction of Target-G vector for PEG-Transfection analysis

In order to evaluate the effects of differences in Target-G vector components on base editing efficiency, vectors for the PEG-Transfection method were constructed. Four types of Target-G units "d11" (SEQ ID NO: 7), "d21" (SEQ ID NO: 8), "n11" (SEQ ID NO: 9), and "n21" (SEQ ID NO: 10) were ligated to the maize ubiquitin gene promoter (SEQ ID NO: 1) and incorporated into the cloning vector pDONR/Zeo (#12535035, ThermoFisher). A guide RNA expression unit "x5" (SEQ ID NO: 22) was incorporated into these to construct the following four types of vectors.
·pOSGd11x5-Zeo
·pOSGd21x5-Zeo
·pOSGn11x5-Zeo
·pOSGn21x5-Zeo

### 1-7. Construction of reporter system for evaluation of Target-G activity

In order to visually detect the editing efficiency of the target DNA sequence by Target-G, two types of GFP reporter systems were constructed. The first type visualizes the accumulation of mutations in the target sequence over time through the disappearance of the GFP signal, and an Enhanced Green Fluorescent Protein (EGFP) expression unit (SEQ ID NO: 27) linked to the cauliflower mosaic virus p35S promoter was utilized. This EGFP expression unit was incorporated into a binary vector for plant transformation (pVS1) together with a hygromycin phosphotransferase (hpt) expression unit (SEQ ID NO: 28) linked to the rice actin gene promoter. This plasmid was named "pRIT4-EGFP". The second type visualizes the editing efficiency of the target DNA sequence by Target-G based on the appearance frequency of the GFP signal. Mutated Green Fluorescent Protein (mGFP) (SEQ ID NO: 29) was constructed by deleting the initiation codon and linking the DNA sequence to which the guide RNA "mGFP switch (SEQ ID NO: 18)" binds, and linked to the downstream of the cauliflower mosaic virus p35S promoter. This was incorporated into a binary vector for plant transformation (pVS1) together with a neomycin phosphotransferase II (nptII) expression unit (SEQ ID NO: 26) linked to the rice actin gene promoter. This plasmid was named "pRIT3-mGFP".

### 2. Introduction of Target-G and evaluation vector into Agrobacterium

The binary vectors "pOSGd11x4", "pOSGd21x4", "pOSGn21x2", "pOSGd13x4", "pOSGd23x4", "pOSGd13-nptIIx6", "pOSGd23-nptIIx6", "pRIT4-EGFP", and "pRIT3-mGFP" prepared as described above were introduced into Agrobacterium tumefaciens strain EHA101 by electroporation (Bio Rad MicroPulser electroporation system).

First, competent cells of Agrobacterium were prepared by the following procedure. The Agrobacterium strain was applied onto an LB agar medium (Bacto Tryptone 10 g/L, Yeast Extract 5 g/L, NaCl 10 g/L, Bacto Agar 15 g (1.5%)) and cultured at 28°C in the dark for 2 days. The obtained single colony was inoculated in 5 mL of a 2xYT liquid medium (Bacto Tryptone 16 g/L, Yeast Extract 10 g/L, NaCl 5 g/L) and cultured with shaking at 28°C in the dark for 12 hr. The suspension (200 µL) was added to 200 mL of 2×YT liquid medium and cultured with shaking at 28°C in the dark to grow to OD600=0.2 - 0.4. Then, bacterial cells were collected by centrifugation (3000 rpm, 4°C, 10 min), suspended in 20 mL of 10 mM HEPES (pH 8.0), and centrifugation was repeated 2-3 times. The bacterial cells collected by centrifugation were suspended in 2 mL of sterile 10% glycerol aqueous solution to prepare competent cells.

Next, each vector was introduced into Agrobacterium by the procedure described below. Each vector was dissolved in sterilized water at a concentration of 1 µg/µL, mixed with 50 µL of the above-mentioned Agrobacterium suspension, transferred to a micropulse cuvette (0.1 cm gap, BioRad), and subjected to electroporation (2.2 kV, 5.8 ms). Then, 800 µL of 2xYT liquid medium was added to this solution, the cells were cultured with shaking at 28°C in the dark for 2 hr, applied onto an LB agar medium containing 100 mg/L spectinomycin, and cultured at 28°C in the dark for 36 - 48 hr. The obtained bacterial colonies were grown in 5 mL of 2xYT liquid medium containing 100 mg/L spectinomycin, dispensed as a glycerol (final concentration 35%) stock into microcentrifuge tubes, and stored at -80°C.

### 3. Introduction of Target-AID evaluation vector into rice cultured cells

Transformation of rice was basically performed according to the method of Terada et al. (2002, DOI:10.1038/nbt737). The detail is shown below.

### 3-1. Preparation of rice callus for transformation

About 100 seeds of rice (Oryza sativa. L Japonica brand; Nihonbare) were dehulled, shaken in 70% ethanol for 1 min, and then immersed in a 2.5% sodium hypochlorite aqueous solution for 20 to 30 min for sterilization. Thereafter, they were rinsed with sterile water and placed on a 2N6 medium (mixed salt for N6 medium (Sigma-Aldrich Co. LLC.) 4.0 g/L, Casamino acid 300 mg/L, Myo-inocitol 100 mg/L, Nicotinic acid 0.5 mg/L, Pyridoxine HCl 0.5 mg/L, Thiamine HCl 0.5 mg/L, L-Proline 2878 mg/L, Sucrose 30.0 g/L, 2,4-D (2,4-dichlorophenoxyacetic acid) 2 mg/L, Gelrite 4.0 g/L, pH 5.8) and cultured at 31.5°C in the dark for 3 weeks to induce dedifferentiated cell aggregate (callus) derived from blastodisc cells. Thereafter, calluses with high cell division activity were selected and used for transformation.

### 3-2. Preparation of Agrobacterium for transformation

Each Agrobacterium solution into which each binary vector was introduced was thawed on ice, 300 µL of which was applied onto an AB medium (NH₄Cl 1 g/L, MgSO₄·7H₂ 0.3 g/L, KCl 0.15 g/L, CaCl₂·2H₂O 0.012 g/L, FeSO₄·7H₂O 0.0025 g/L, K₂HPO₄ 3 g/L, NaH₂PO₄·H₂O 1.15 g/L, Sucrose 5.5 g/L, Agarose 6.0 g/L, pH 7.2) supplemented with 100 mg/L spectinomycin, and cultured at 28°C in the dark for 3 days. Thereafter, the proliferated Agrobacterium was suspended in AAI liquid medium (MgSO₄·7H₂O 5 g/L, CaCl₂·2H₂O 1.5 g/L, NaH₂PO₄·H₂O 1.5 g/L, KCl 29.5 g/L, MnSO₄·4H₂O 10 g/L, ZnSO₄·7H₂O 2 g/L, H₃BO₃ 3 g/L, KI 0.75 g/L, Na₂MoO₄·2H₂O 0.25 g/L, CoCl₂·6H₂O 25 mg/L, CuSO₄·5H₂O 25 mg/L, FeSO₄·7H₂O 13.9 g/L, Na₂ EDTA 18.7 g/L, Myo-inocitol 100 mg/L, Thiamine HCl 0.01 g/L, Nicotinic acid 1 mg/L, Pyridoxine HCl 1 mg/L) supplemented with 40 mg/L acetosyringone (3',5'-Dimethoxy-4'-hydroxy-acetophenone) and cultured with shaking at 25°C for 2 hr. This suspension was diluted with AAI liquid medium containing 40 mg/L Acetosyringone to prepare 120 ml of suspension with OD600=0.008.

### 3-3. Introduction of Target-G vector into rice callus (Agrobacterium inoculation, coculture, sterilization, rice recombinant callus selection)

About 15 g of the rice calluses obtained in 3-1 were collected in a sterilized glass beaker, and the Agrobacterium suspension containing each vector (described above) was added and inoculated with shaking for 3 to 5 min. Thereafter, the suspension was filtered through a stainless mesh (1.5 mm joint opening) to remove excess Agrobacterium. Next, a sterilized filter paper was put on top of the 2N6 co-culture medium (40 mg/L of acetosyringone added to the above-mentioned 2N6 medium, pH 5.2), arranged the calluses thereon with tweezers at equal intervals, and co-cultured for 3 days at 25°C in the dark. Thereafter, in order to sterilize the calluses after co-cultivation from the Agrobacterium, the calluses were collected in a 500 ml beaker, and washed with sterilization solution 1 (sterilized water containing 200 mg/L Vancomycin and 20 µl/L Tween 20) for 30 min with stirring. Thereafter, the callused were collected on a stainless steel mesh, the water around the callus was removed with a paper towel, and the same sterilization operation was repeated four times using 300 ml of sterilization solution 2 (sterilized water containing 200 mg/L of vancomycin). Then, the sterilized calluses were cultured for 5 days in 2N6NU medium (2N6 medium supplemented with 100 mg/L of vancomycin and 25 mg/L of Meropenem, pH 5.8). Thereafter, they were arranged at equal intervals on the selective medium 2N6SE H40 (2N6NU medium with 40 mg/L of hygromycin added) or 2N6SE Pa50 (2N6NU medium with 50 mg/L of paromomycin added), and cultured for about 4 to 6 weeks at 31.5°C in the dark.

### 3-4. Confirmation of mutation introduction

Genomic DNA was extracted from Hygromycin-resistant callus by using an automatic nucleic acid extractor (PX-80, Kurabo Industries, Ltd.). The target gene region of each callus was amplified by PCR method, and then direct sequence analysis was performed to confirm introduction of mutation by Target-G. Furthermore, PCR products obtained from some of the calluses were subjected to cloning sequence analysis to confirm the introduction of mutations in detail.

### 3-5. Transient expression analysis using rice cultured cell

The liquid medium is removed from the rice cultured cells cultured with shaking in the liquid medium, an enzyme solution (Cellulase 1.6 g, Macerozyme 0.4 g, Sucrose 6.86 g, CaCl₂ 2H₂O 58.8 mg/L, MES 40 mg, BSA 40 mg, diluted to 40 ml with sterilized water) is added, and the cells are treated at 30°C for about 6 hr. Thereafter, a wash buffer (KCl 373 mg, NaCl 9 g, CaCl₂ 2H₂O 18.4 mg, MES 427 mg, diluted to 40 ml with sterilized water) is added, protoplasts are separated by centrifugation, aspirated with a pipette and transferred to a new container. Thereafter, the cells are suspended in a wash buffer, centrifuged, and the supernatant is discarded. This step is repeated for washing. After mixing the plasmid DNA to be introduced and the protoplasts in a microcentrifuge tube, a PEG solution (PEG4000 2 g, 0.8 M Mannitol 1.25 ml, 1 M CaCl₂ 0.5 ml, diluted to 5 ml with sterilized water) is added. After incubation at 30°C for 24 - 72 hr, the protoplasts were made into a preparation and analyzed using a fluorescence microscope.

### (Example 2: Introduction of target mutation by Target-G system using nCas9)

In order to evaluate the target gene modification of monocotyledonous plants by the Target-G system using nCas9, "pOSGn21x2" was introduced into rice callus by the Agrobacterium method. As a result, only 3 lineages out of 323 lineages showed hygromycin resistance, and the transformation efficiency was 1.28%. Genomic DNA was extracted from these calluses, and the introduction of mutations in the target regions (two sites in the ALS gene) was confirmed. As a result, the frequency of large-scale deletions was higher than that of base substitution mutations (Fig. 2). The reason therefor was assumed to be that when nCas9 is used in the Target-G system, the repair process of the abasic site induced by UNG and the DNA single-strand breakage by nCas9 proceed simultaneously, resulting in the occurrence of DNA double-strand breaks (DSB) at high frequency.

### (Example 3: Introduction of target mutation by Target-G system using dCas9)

In order to solve the above problems, target gene modification by Target-G system using dCas9 was attempted. "pOSGd11x4" and "pOSGd21x4" were simultaneously transformed into rice callus. As a result, 54 lineages out of 1364 lineages of callus showed hygromycin resistance, and the transformation efficiency was 3.96%. Direct sequence analysis confirmed the introduction of mutations into four target genes (ALS, OsFTIP1e, DL, OsClpP5) in multiple hygromycin-resistant callus lineages (Figs. 3 to 6). In addition, multiple target genes were modified within one callus, thus demonstrating simultaneous multipoint editing by Target-G. Subsequently, cloning sequence analysis was performed on some callus lineages, and as a result, point mutations and deletion/insertion mutations occurred somewhat randomly around the target sequence (Figs. 7 to 10). The main mutations were point mutations from C to G, as demonstrated using Saccharomyces cerevisiae in the basic patent of the present technique (WO 2016072399). This suggests that the genome editing technique of the present invention is suitable for use in randomly introducing mutations into and around target nucleotide sequences in plant genomes to promote local evolution.

### (Example 4: Redifferentiation of T0 plants and transmission of mutations (from callus to T0 plant))

T0 plants were redifferentiated from multiple lineages of callus in which mutation introduction into the target gene was confirmed, and the status of mutation transfer was investigated. As a result of direct sequencing analysis, it was confirmed that the mutations identified in callus were transferred to T0 plants (Figs. 11 to 13). Moreover, while T0 plants derived from the same callus share the same mutation, mutations unique to each plant were also confirmed (Figs. 12 and 13). Furthermore, introduction of Biallelic mutation was also confirmed (Fig. 11).

### (Example 5: Inheritance and segregation of introduced mutations to the T1 generation, expansion of further mutations)

The T1 generation plant body obtained by self-reproduction of the T0 plant body was developed. As a result, some plants exhibiting an albino phenotype were isolated (Fig. 14). As a result of analyzing the genome sequences of them, mutations in OsClpP5 were confirmed. Similarly, plants exhibiting a "weeping leaf" phenotype were isolated, and mutations in the DL gene were confirmed in them. In addition, T1 plants derived from the same T0 plant were subjected to PCR analysis, and classified into those that possessed the Target-G expression unit and those that did not. The sequences of four target genes (ALS, OsFTIP1e, DL, OsClpP5) in those plants were confirmed. As a result, more diverse mutations occurred in the vector-carrying plants. This confirms that Target-G accumulates mutations in the target region on the genome over time.

### (Example 6: Examination of optimal configuration of Target-G system)

In Example 2, when the constitutively expressed Target-G was introduced into rice callus, the transformation efficiency was as low as 3.96%. Therefore, the configuration of the Target-G system with low lethality and high editing efficiency was investigated. First, rice callus introduced with a reporter system "pRIT3-mGFP" that detects base substitutions in the target region was prepared and transformed into liquid culture cells. Next, four types of vectors "pOSGd11x5-Zeo" "pOSGd21x5-Zeo" "pOSGn11x5-Zeo", and "pOSGn21x5-Zeo" were introduced by PEG-Transfection. Protoplasts after 24-72 hr were observed with a fluorescence microscope. As a result, GFP signal was confirmed only in "pOSGd11x5-Zeo", which suggests that a unit in which one copy of UNG is linked to dCas9 is suitable for introducing base substitutions while reducing DSBs in the target region. Subsequently, genomic DNA was extracted from the plasmid into which each vector had been introduced, and mutation introduction in the five target regions (ALS, OsFTIP1e, DL, OsClpP5, mGFP) was analyzed by deep sequence analysis. As a result, when nCas9 was used, deletions and insertions were confirmed at high frequency in the target region in the Target-G system, thus suggesting the occurrence of DNA double-strand break (DSB). When dCas9 was used, while the frequency of deletion·insertion was suppressed, various base substitution mutations were confirmed. In addition, the frequency of deletion·insertion was clearly lower in the case of 1-copy UNG than in the case of 2-copy UNG. From the above, it was determined that the optimal configuration of the Target-G system is one copy of UNG ligated to dCas9.

### (Example 7: Evaluation of inducible type Target-G system)

To avoid lethality by Target-G, a heat shock-induced Target-G system was constructed and the controllability and target sequence editing efficiency thereof were evaluated. First, the reporter system "pRIT4-EGFP" was introduced into rice callus by the Agrobacterium method to prepare rice callus for evaluation of editing efficiency. Then, "pOSGd13-nptIIx6" or "pOSGd23-nptIIx6" was introduced into the callus that constitutively expresses EGFP to obtain a double transformed callus resistant to hygromycin and paromomycin. Each callus was isolated, proliferated as an independent lineage, and then divided into 5 equal parts. Thereafter, a heat shock treatment (42°C, 90 min) was performed every 5 days, and the accumulation of mutations depending on the number of treatments was investigated. After 1 to 4 times of heat shock treatments, the cells were cultured for 3 weeks, and genomic DNA was extracted from each callus and subjected to deep sequence analysis. Analysis of mutations in six target regions (ALS, OsFTIP1e, DL, OsClpP5, EGFP-1, EGFP-2) showed that the activity of Target-G was suppressed at general culture temperature (31.5°C). On the other hand, it was confirmed that the accumulation of mutations in each target region increases with the number of heat shock treatments.

### (Example 8: Introduction of target mutation by inducible type Target-G system)

Based on the results obtained in Example 7, a rice target gene plant was produced using the inducible type Target-G system. "pOSGd13x4" or "pOSGd23x4" was introduced into the rice callus by the Agrobacterium method. As a result, the transformation efficiency was improved, and the browning necrosis of each transformed callus could be avoided as compared with the introduction of the constitutively expressing Target-G. This suggests that the lethality of Target-G can be reduced by using an inducible type promoter. Then, each transformed callus lineage obtained was subjected to a heat shock treatment (42°C, 90 min) every 5 days for a total of 4 times, and after culturing for 2 weeks, T0 plant body was redifferentiated. Genomic DNA was extracted from each T0 plant body, and deep sequence analysis was performed to analyze the status of mutation introduction in the four target regions (ALS, OsFTIP1e, DL, and OsClpP5). As a result, the accumulation of mutations was confirmed. Furthermore, more diverse mutations were confirmed than in the constitutive expression type. The reason therefor is assumed to be that the number of surviving cells increased and mutations in their genomes could also be detected. In addition, an improvement in seed fertility was observed, which is assumed to be because excessive KO mutations in the DL gene could be avoided.

### (Note)

As described above, the present disclosure is exemplified by the use of its preferred embodiments. However, it is understood that the scope of the present disclosure should be interpreted solely based on the Claims. It is also understood that any patent, any patent application, and any references cited herein should be incorporated herein by reference in the same manner as the contents are specifically described herein.

### [Industrial Applicability]

The methods of the present disclosure allow artificial manipulation of gene function in any organism, including animals, plants, microorganisms, and the like. Therefore, a wide range of applications such as medical applications, development of agricultural products, modification of industrial microorganisms, and the like are expected.

[Sequence Listing Free Text]

SEQ ID NO: 1: maiz ubiquitin gene promoter
SEQ ID NO: 2: nuclease completely inactivated Cas9 (dCas9) gene
SEQ ID NO: 3: dCas9 N terminal side nuclear localization signal
SEQ ID NO: 4: dCas9 C-terminal side nuclear localization signal
SEQ ID NO: 5: nuclease partially inactivated Cas9 (nCas9) gene
SEQ ID NO: 6: modified type UNG1 gene
SEQ ID NO: 7: dCas9-UNG1 expression unit (d1)
SEQ ID NO: 8: dCas9-UNG1 expression unit (d2)
SEQ ID NO: 9: nCas9-UNG1 expression unit (n1)
SEQ ID NO: 10: nCas9-UNG1 expression unit (n2)
SEQ ID NO: 11: rice RNA polymerase III dependent U6 promoter
SEQ ID NO: 12: guide RNA: ALS A96
SEQ ID NO: 13: guide RNA: OsFTIP1e Q590
SEQ ID NO: 14: guide RNA: DL-3
SEQ ID NO: 15: guide RNA: OsClpP5
SEQ ID NO: 16: guide RNA: EGFP Mut1
SEQ ID NO: 17: guide RNA: EGFP Mut2
SEQ ID NO: 18: guide RNA: mGFP switch
SEQ ID NO: 19: guide RNA: ALS P171-R173
SEQ ID NO: 20: guide RNA unitx4
SEQ ID NO: 21: guide RNA unitx6
SEQ ID NO: 22: guide RNA unitx5
SEQ ID NO: 23: guide RNA unitx2
SEQ ID NO: 24: hygromycin phosphotransferase (hpt) expression
unit
SEQ ID NO: 25: rice heat shock protein 17.9C gene promoter
SEQ ID NO: 26: neomycin phosphotransferase II (nptII)
expression unit
SEQ ID NO: 27: Enhanced Green Fluorescent Protein (EGFP)
expression unit
SEQ ID NO: 28: hygromycin phosphotransferase (hpt) expression
unit

SEQ ID NO: 29: mutated Green Fluorescent Protein (mGFP) gene

## Claims

1. A method for producing a plant cell modified at a targeted site of a double-stranded DNA, comprising
(i) a step of providing a plant cell comprising the double-stranded DNA of interest,
(ii) a step of providing a complex in which a nucleic acid sequence-recognizing module that specifically binds to a target nucleotide sequence in the double-stranded DNA and a DNA glycosylase with sufficiently low reactivity with the double-stranded DNA are bound,
(iii) a step of placing the complex in a condition under which the plant cell is transfected,
(iv) a step of placing the transfected plant cell in a condition that induces modification of the targeted site, without cleaving at least one strand of the double-stranded DNA in the targeted site, and
(v) a step of selecting a cell into which the complex has been introduced and/or a cell into which the modification has been introduced.

2. The method according to claim 1, wherein the nucleic acid sequence-recognizing module is selected from the group consisting of a CRISPR-Cas system wherein at least one DNA cleavage ability of Cas is inactivated, a zinc finger motif, a TAL effector and a PPR motif.

3. The method according to claim 1 or 2, wherein the nucleic acid sequence-recognizing module is a CRISPR-Cas system in which Cas does not have at least one DNA cleavage ability.

4. The method according to claim 1 or 2, wherein the nucleic acid sequence-recognizing module is a CRISPR-Cas system in which Cas does not have both of DNA cleavage ability.

5. The method according to any one of claims 1 to 4, wherein the modification comprises substitution or deletion of one or more nucleotides in the targeted site, or insertion of one or more nucleotides in the targeted site.

6. The method according to any one of claims 1 to 5, wherein the modification dominantly occurs on the PAM sequence side of the targeted site.

7. The method according to any one of claims 1 to 6, wherein the DNA glycosylase is a mutant whose reactivity with double-stranded DNA is attenuated as compared with the wild type.

8. The method according to any one of claims 1 to 7, wherein the DNA glycosylase has cytosine-DNA glycosylase (CDG) activity or thymine-DNA glycosylase (TDG) activity.

9. The method according to claim 8, wherein the DNA glycosylase having CDG activity or TDG activity is a mutant of uracil-DNA glycosylase (UDG).

10. The method according to any one of claims 1 to 9, wherein the DNA glycosylase is a mutant of uracil-DNA glycosylase (UDG) derived from a yeast and having CDG activity or TDG activity.

11. The method according to any one of claims 1 to 10,
wherein the plant cell is derived from rice, Arabidopsis thaliana, bean, maize, cotton, safflower, sunflower, tobacco, wheat, barley, hemp, rose, Japanese yew, banana, coffee, sesame, buckwheat, or lettuce.

12. The method according to any one of claims 1 to 11, wherein the plant cell is derived from rice or Arabidopsis thaliana.

13. The method according to any one of claims 1 to 12, wherein the transfection is performed through delivery of the complex to separated plant callus or by the Floral dip method.

14. The method according to claim 13, wherein the delivery is performed by the Agrobacterium method.

15. The method according to any one of claims 1 to 14, further comprising a step of producing a plant body from the cell.

16. The method according to any one of claims 1 to 15, further comprising a step of clonally separating the obtained cell.

17. A transformed plant cell obtainable by the method according to any one of claims 1 to 16.

18. A transformed plant comprising the plant cell according to claim 17.

19. A seed obtained from the plant according to claim 18.

20. The plant according to claim 18, wherein the transformed trait is expressed only in the primary transgenic generation.

21. The plant according to claim 18, wherein the expression of the transformed trait is inherited across generations.

22. A method for producing a cell modified at a targeted site of a double-stranded DNA, comprising
(i) a step of providing a cell comprising the double-stranded DNA of interest,
(ii) a step of providing a complex in which a nucleic acid sequence-recognizing module that specifically binds to a target nucleotide sequence in the double-stranded DNA and a DNA glycosylase with sufficiently low reactivity with the double-stranded DNA are bound,
(iii) a step of placing the complex in a condition under which the cell is transfected,
(iv) a step of placing the transfected cell in a condition that induces modification of the targeted site, without cleaving at least one strand of the double-stranded DNA in the targeted site, and
(v) a step of selecting a cell into which the complex has been introduced and/or a cell into which the modification has been introduced, wherein
the modification in the cell is maintained for at least the primary transformant.

23. A method for producing a plant cell having a desired property, comprising
(i) a step of providing a plant cell comprising a double-stranded DNA related to the desired property,
(ii) a step of providing a complex in which a nucleic acid sequence-recognizing module that specifically binds to a target nucleotide sequence in the double-stranded DNA and a DNA glycosylase with sufficiently low reactivity with the double-stranded DNA are bound,
(iii) a step of placing the complex in a condition under which the plant cell is transfected,
(iv) a step of placing the transfected plant cell in a condition that induces modification of the targeted site, without cleaving at least one strand of the double-stranded DNA in the targeted site,
(v) a step of selecting a cell into which the complex has been introduced and/or a cell into which the modification has been introduced, and
(vi) a step of selecting the cell having the desired property from the introduced cells.
